# EUROPEAN PATENT APPLICATION

(11) **EP 4 245 172 A1**
(43) Date of publication of application: **20.09.2023**
(21) Application number: 20961483.3
(22) Date of filing: 10.11.2020
(51) Int. Cl.: A24F 40/50

(54) **INFORMATION PROCESSING DEVICE, INFORMATION PROCESSING METHOD, AND PROGRAM**

(71) Applicant: Japan Tobacco Inc., Tokyo 105-6927 (JP)
(72) Inventor: YAMADA, Manabu, Tokyo 130-8603 (JP)
(74) Representative: Hoffmann Eitle
(86) International application number: PCT/JP2020/041796
(87) International publication number: WO 2022/101955

(57) **Abstract**

[Problem] To provide a mechanism which enables a user to more easily customize the operation of an inhalation device.

[Solution] An information processing device provided with a control part, wherein
the control part: generates a display image for displaying a profile that is information indicating time series variation in a parameter regarding an operation for generating an aerosol, the operation being performed by an inhalation device for generating the aerosol by using a base material; and modifies the profile and the display of the profile in the display image in accordance with the user operation with respect to the parameter to be operated in the generated display image.

## Description

### Technical Field

The present invention relates to an information processing device, an information processing method, and a program.

### Background Art

Inhaler devices, such as e-cigarettes and nebulizers, for generating a substance to be inhaled by a user are widespread. For example, an inhaler device generates an aerosol to which a flavor component is imparted, by using a substrate including an aerosol source for generating the aerosol, a flavor source for imparting the flavor component to the generated aerosol, and the like. The user can enjoy the flavor by inhaling the aerosol to which the flavor component is imparted, which is generated by the inhaler device.

In recent years, various technologies related to inhaler devices have been developed to provide more enriched inhalation experiences to users. For example, Patent Literature 1 below discloses a technique for changing the amount of aerosol to be delivered by an inhaler device over time.

### Citation List

### Patent Literature

Patent Literature 1: WO2020/084776

### Summary of Invention

### Technical Problem

However, the technique disclosed in Patent Literature 1 above has not been developed to its full potential, and further technical development for providing a more enriched inhalation experience to the user is being demanded.

In order to solve the above problem, the present invention provides a mechanism that allows a user to more easily customize the operation of an inhaler device.

### Solution to Problem

In order to solve the above problem, according to an aspect of the present invention, there is provided an information processing device including a controller configured to generate a display image that displays a profile, the profile being information indicating a time-series change of a parameter related to an operation of generating an aerosol, the operation being performed by an inhaler device configured to generate the aerosol by using a substrate; and change the profile and display of the profile in the display image in accordance with a user operation on the parameter to be operated in the generated display image.

The controller may be configured to change the parameter to be operated in the profile in accordance with the user operation of changing the parameter to be operated.

The display image may include information indicating that the parameter to be operated is changeable.

The display image may include information indicating a range in which the parameter to be operated is changeable.

The controller may be configured to change a range in which the parameter to be operated is changeable, in response to another parameter to be operated being changed in accordance with the user operation.

The parameter to be operated may include the parameter at a puff detection time, the puff detection time being a time at which inhalation of the aerosol generated by the inhaler device is detected, the inhalation being performed by a user

The parameter to be operated may include the parameter at a time other than a puff detection time, the puff detection time being a time at which inhalation of the aerosol generated by the inhaler device is detected, the inhalation being performed by a user

The display image may display the parameter to be operated in a mode different from a mode of a parameter that is not to be operated.

The display image may display the profile from a start time point to an end time point in association with information indicating the parameter to be operated from the start time point to the end time point.

The display image may display the profile from a start time point to an end time point in association with information indicating the parameter to be operated from the start time point to a current time point.

The display image may display the profile from a start time point to a current point in association with information indicating the parameter to be operated from the start time point to the current time point.

The display image may display an extracted portion of the profile in part of an entire time section from a start time point to an end time point, the part including a puff detection time, the puff detection time being a time at which inhalation of the aerosol generated by the inhaler device is detected, the inhalation being performed by a user

The display image may further display another profile to be compared with the profile.

The display image may display, as the profile, a difference between the profile and another profile serving as a reference.

The other profile may be the profile on which no change has been made.

The profile may be a first profile including, as the parameter, information that defines an operation in which the inhaler device generates the aerosol.

The controller may be configured to control the inhaler device so that the inhaler device operates in accordance with the first profile on which a change has been made.

The display image may further include information predicted to be detected in response to the inhaler device operating in accordance with the first profile on which a change has been made.

The profile may be a second profile including, as the parameter, information detected in response to the inhaler device performing the operation of generating the aerosol.

The controller may be configured to generate a first profile including, as a parameter, information that defines an operation in which the inhaler device generates the aerosol to implement the second profile on which a change has been made, and control the inhaler device so that the inhaler device operates in accordance with the generated first profile.

The display image may display the profile as a graph.

The display image may display, as a table, the parameter to be operated.

In order to solve the above problem, according to another embodiment of the present invention, there is provided an information processing method including generating a display image that displays a profile, the profile being information indicating a time-series change of a parameter related to an operation of generating an aerosol, the operation being performed by an inhaler device configured to generate the aerosol by using a substrate; and changing the profile and display of the profile in the display image in accordance with a user operation on the parameter to be operated in the generated display image.

In order to solve the above problem, according to another embodiment of the present invention, there is provided a program for causing a computer to function as a controller configured to generate a display image that displays a profile, the profile being information indicating a time-series change of a parameter related to an operation of generating an aerosol, the operation being performed by an inhaler device configured to generate the aerosol by using a substrate; and change the profile and display of the profile in the display image in accordance with a user operation on the parameter to be operated in the generated display image.

### Advantageous Effects of Invention

As described above, according to the present invention, there is provided a mechanism that allows a user to more easily customize the operation of an inhaler device.

### Brief Description of Drawings

[Fig. 1] Fig. 1 is a schematic diagram of an inhaler device according to a first configuration example.
[Fig. 2] Fig. 2 is a schematic diagram of an inhaler device according to a second configuration example.
[Fig. 3] Fig. 3 is a diagram illustrating an example of the configuration of a system according to the present embodiment.
[Fig. 4] Fig. 4 is a graph illustrating an example of a heating profile according to the present embodiment.
[Fig. 5] Fig. 5 is a diagram illustrating an example of a display image generated by a user terminal according to the present embodiment.
[Fig. 6] Fig. 6 is a diagram illustrating an example of the display image generated by the user terminal according to the present embodiment.
[Fig. 7] Fig. 7 is a diagram illustrating an example of the display image generated by the user terminal according to the present embodiment.
[Fig. 8] Fig. 8 is a diagram illustrating an example of the display image generated by the user terminal according to the present embodiment.
[Fig. 9] Fig. 9 is a diagram illustrating an example of the display image generated by the user terminal according to the present embodiment.
[Fig. 10] Fig. 10 is a diagram illustrating an example of the display image generated by the user terminal according to the present embodiment.
[Fig. 11] Fig. 11 is a diagram illustrating an example of the display image generated by the user terminal according to the present embodiment.
[Fig. 12] Fig. 12 is a diagram illustrating an example of the display image generated by the user terminal according to the present embodiment.
[Fig. 13] Fig. 13 is a diagram illustrating an example of the display image generated by the user terminal according to the present embodiment.
[Fig. 14] Fig. 14 is a diagram illustrating an example of the display image generated by the user terminal according to the present embodiment.
[Fig. 15] Fig. 15 is a diagram illustrating an example of the display image generated by the user terminal according to the present embodiment.
[Fig. 16] Fig. 16 is a diagram illustrating an example of the display image generated by the user terminal according to the present embodiment.
[Fig. 17] Fig. 17 is a diagram illustrating an example of the display image generated by the user terminal according to the present embodiment.
[Fig. 18] Fig. 18 is a diagram illustrating an example of the display image generated by the user terminal according to the present embodiment.
[Fig. 19] Fig. 19 is a diagram illustrating an example of the display image generated by the user terminal according to the present embodiment.
[Fig. 20] Fig. 20 is a diagram illustrating an example of the display image generated by the user terminal according to the present embodiment.
[Fig. 21] Fig. 21 is a diagram illustrating an example of the display image generated by the user terminal according to the present embodiment.
[Fig. 22] Fig. 22 is a diagram illustrating an example of the display image generated by the user terminal according to the present embodiment.
[Fig. 23] Fig. 23 is a diagram illustrating an example of the display image generated by the user terminal according to the present embodiment.
[Fig. 24] Fig. 24 is a diagram illustrating an example of the display image generated by the user terminal according to the present embodiment.
[Fig. 25] Fig. 25 is a diagram illustrating an example of the display image generated by the user terminal according to the present embodiment.
[Fig. 26] Fig. 26 is a diagram illustrating an example of the display image generated by the user terminal according to the present embodiment.
[Fig. 27] Fig. 27 is a diagram illustrating an example of the display image generated by the user terminal according to the present embodiment.
[Fig. 28] Fig. 28 is a diagram illustrating an example of the display image generated by the user terminal according to the present embodiment.
[Fig. 29] Fig. 29 is a diagram illustrating an example of the display image generated by the user terminal according to the present embodiment.
[Fig. 30] Fig. 30 is a diagram illustrating an example of the display image generated by the user terminal according to the present embodiment.
[Fig. 31] Fig. 31 is a diagram illustrating an example of the display image generated by the user terminal according to the present embodiment.
[Fig. 32] Fig. 32 is a diagram illustrating an example of the display image generated by the user terminal according to the present embodiment.
[Fig. 33] Fig. 33 is a diagram illustrating an example of the display image generated by the user terminal according to the present embodiment.
[Fig. 34] Fig. 34 is a sequence diagram illustrating an example of a process flow executed in the system according to the present embodiment.
[Fig. 35] Fig. 35 is a flowchart illustrating an example of a process flow executed in the system according to the present embodiment.

### Description of Embodiments

A preferred embodiment of the present invention will be described in detail hereinafter with reference to the accompanying drawings. In the specification and the drawings, structural elements having substantially the same functional configuration are denoted by the same reference numerals, and redundant description thereof will be omitted.

### «1. Configuration example of inhaler device»

An inhaler device generates material to be inhaled by a user. In the example described below, the material generated by the inhaler device is an aerosol. Alternatively, the material generated by the inhaler device may be gas.

### (1) First configuration example

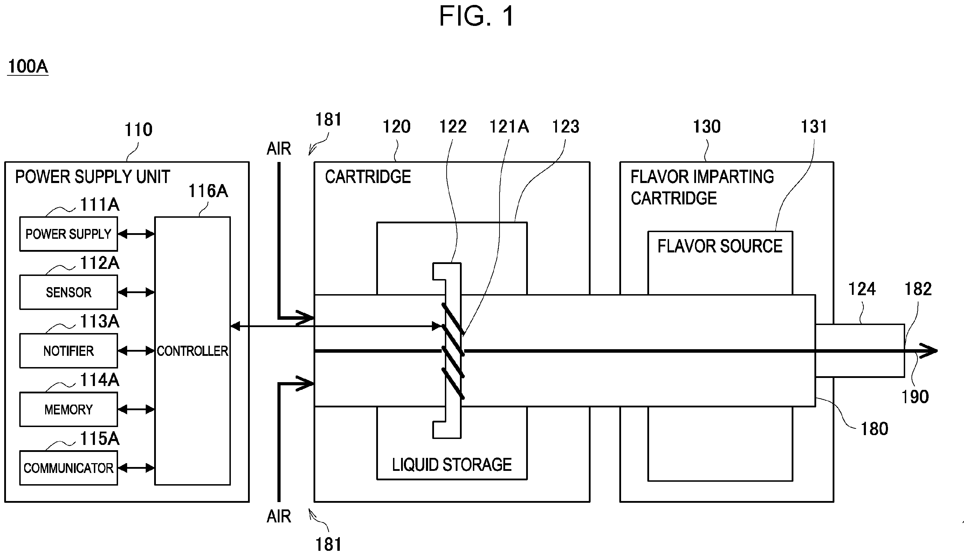

Fig. 1 is a schematic diagram of the inhaler device according to the first configuration example. As illustrated in Fig. 1, an inhaler device 100A according to the present configuration example includes a power supply unit 110, a cartridge 120, and a flavor imparting cartridge 130. The power supply unit 110 includes a power supply 111A, a sensor 112A, a notifier 113A, a memory 114A, a communicator 115A, and a controller 116A. The cartridge 120 includes a heater 121A, a liquid guide 122, and a liquid storage 123. The flavor imparting cartridge 130 includes a flavor source 131 and a mouthpiece 124. In the cartridge 120 and the flavor imparting cartridge 130, an airflow path 180 is defined.

The power supply 111A stores electric power. The power supply 111A supplies electric power to the structural elements of the inhaler device 100A under the control of the controller 116A. The power supply 111A may be a rechargeable battery such as a lithium ion secondary battery.

The sensor 112A acquires various items of information regarding the inhaler device 100A. In an example, the sensor 112A may be a pressure sensor such as a condenser microphone, a flow sensor, or a temperature sensor, and acquire a value generated in accordance with the user's inhalation. In another example, the sensor 112A may be an input device that receives information input by the user, such as a button or a switch.

The notifier 113A provides information to the user. The notifier 113A may be a light-emitting device that emits light, a display device that displays an image, a sound output device that outputs sound, or a vibration device that vibrates.

The memory 114A stores various items of information for operation of the inhaler device 100A. The memory 114A may be a non-volatile storage medium such as flash memory.

The communicator 115A is a communication interface capable of communication in conformity with any wired or wireless communication standard. Such a communication standard may be, for example, Wi-Fi (registered trademark) or Bluetooth (registered trademark).

The controller 116A functions as an arithmetic processing unit and a control circuit, and controls the overall operations of the inhaler device 100A in accordance with various programs. The controller 116A includes an electronic circuit such as a central processing unit (CPU) and a microprocessor, for example.

The liquid storage 123 stores an aerosol source. The aerosol source is atomized to generate an aerosol. The aerosol source is a liquid such as polyhydric alcohol and water. Examples of the polyhydric alcohol include glycerine and propylene glycol. The aerosol source may include a flavor component that is either derived from tobacco or not derived from tobacco. For the inhaler device 100A that is a medical inhaler such as a nebulizer, the aerosol source may include a medicine.

The liquid guide 122 guides, from the liquid storage 123, the aerosol source that is the liquid stored in the liquid storage 123, and holds the aerosol source. The liquid guide 122 is, for example, a wick formed by twining fiber material such as glass fiber or porous material such as porous ceramic. In this case, the capillary action of the wick guides the aerosol source stored in the liquid storage 123.

The heater 121A heats the aerosol source to atomize the aerosol source and generate the aerosol. In the example illustrated in Fig. 1, the heater 121A includes a coil wound around the liquid guide 122. When the heater 121A produces heat, the aerosol source held by the liquid guide 122 is heated and atomized to generate the aerosol. The heater 121A produces heat when receiving electric power from the power supply 111A. In an example, the electric power may be supplied in response to the sensor 112A detecting a start of the user's inhalation and/or an input of predetermined information. Subsequently, the supply of the electric power may be stopped in response to the sensor 112A detecting an end of the user's inhalation and/or an input of predetermined information.

The flavor source 131 is a structural element for imparting a flavor component to the aerosol. The flavor source 131 may include a flavor component that is either derived from tobacco or not derived from tobacco.

The airflow path 180 is a flow path of air to be inhaled by the user. The airflow path 180 has a tubular structure having an air inlet hole 181 and an air outlet hole 182 at both ends. The air inlet hole 181 is an inlet of air into the airflow path 180, and the air outlet hole 182 is an outlet of the air from the airflow path 180. The liquid guide 122 is on the airflow path 180 at an upstream position (closer to the air inlet hole 181), and the flavor source 131 is on the airflow path 180 at a downstream position (closer to the air outlet hole 182). Air flowing in through the air inlet hole 181 when the user inhales mixes with the aerosol generated by the heater 121A. Subsequently, as indicated by an arrow 190, the mixture fluid of the aerosol and the air passes through the flavor source 131 and is conveyed to the air outlet hole 182. When the mixture fluid of the aerosol and the air passes through the flavor source 131, the flavor component included in the flavor source 131 is imparted to the aerosol.

The mouthpiece 124 is to be held in a mouth of the user during inhalation. The mouthpiece 124 has the air outlet hole 182. When the user inhales with the mouthpiece 124 in his/her mouth, the mixture fluid of the aerosol and the air enters the oral cavity of the user.

The configuration example of the inhaler device 100A has been described above. The inhaler device 100A is not limited to the above configuration, and may be configured in various ways as exemplified below.

In an example, the inhaler device 100A does not have to include the flavor imparting cartridge 130. In this case, the cartridge 120 includes the mouthpiece 124.

In another example, the inhaler device 100A may include various types of aerosol sources. Still another type of aerosol may be generated by mixing a plurality of types of aerosols generated from the plurality of types of aerosol sources in the airflow path 180 and causing a chemical reaction.

In addition, means for atomizing the aerosol source is not limited to heating by the heater 121A. For example, the means for atomizing the aerosol source may be vibration atomization or induction heating.

### (2) Second configuration example

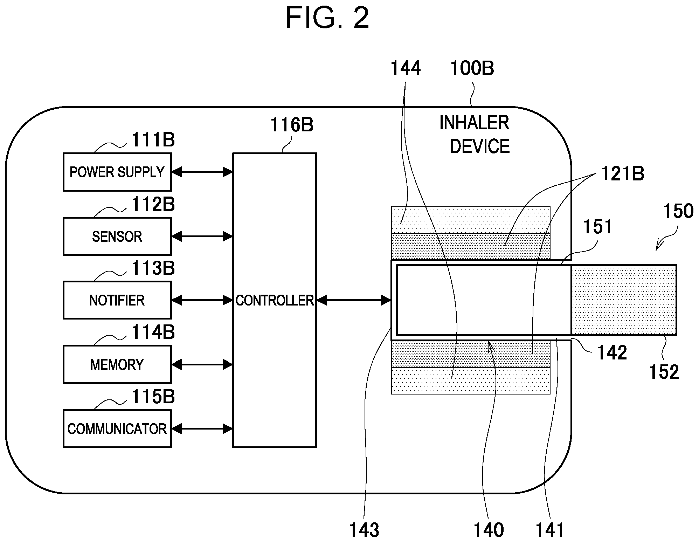

Fig. 2 is a schematic diagram of the inhaler device according to the second configuration example. As illustrated in Fig. 2, an inhaler device 100B according to the present configuration example includes a power supply 111B, a sensor 112B, a notifier 113B, a memory 114B, a communicator 115B, a controller 116B, a heater 121B, a holder 140, and a heat insulator 144.

The power supply 111B, the sensor 112B, the notifier 113B, the memory 114B, the communicator 115B, and the controller 116B are substantially the same as the respective corresponding structural elements included in the inhaler device 100A according to the first configuration example.

The holder 140 has an internal space 141, and holds a stick substrate 150 in a manner partially accommodated in the internal space 141. The holder 140 has an opening 142 that allows the internal space 141 to communicate with outside. The holder 140 accommodates the stick substrate 150 that is inserted into the internal space 141 through the opening 142. For example, the holder 140 may be a tubular body having the opening 142 and a bottom 143 on its ends, and may define the pillar-shaped internal space 141. The holder 140 also has a function of defining a flow path of air to be supplied to the stick substrate 150. An air inlet hole, which is an inlet of air to the flow path, is disposed in the bottom 143, for example. An air outlet hole, which is an outlet of air from the flow path, is the opening 142.

The stick substrate 150 includes a substrate 151 and an inhalation port 152. The substrate 151 includes an aerosol source. In the present configuration example, the aerosol source is not limited to a liquid, and may be a solid. The stick substrate 150 held by the holder 140 includes the substrate 151 at least partially accommodated in the internal space 141 and the inhalation port 152 at least partially protruding from the opening 142. When the user inhales with the inhalation port 152 protruding from the opening 142 in his/her mouth, air flows into the internal space 141 from the air inlet hole (not illustrated), and the air and an aerosol generated from the substrate 151 reach inside the mouth of the user.

The heater 121B has a configuration similar to that of the heater 121A according to the first configuration example. In the example illustrated in Fig. 2, the heater 121B has a film-like shape and surrounds the outer circumference of the holder 140. Subsequently, heat produced from the heater 121B heats the substrate 151 of the stick substrate 150 from the outer circumference, generating the aerosol.

The heat insulator 144 prevents heat from transferring from the heater 121B to the other structural elements. For example, the heat insulator 144 may be a vacuum heat insulator or an aerogel heat insulator.

The configuration example of the inhaler device 100B has been described above. The inhaler device 100B is not limited to the above configuration, and may be configured in various ways as exemplified below.

In an example, the heater 121B may have a blade-like shape, and may be disposed so that the heater 121B protrudes from the bottom 143 of the holder 140 toward the internal space 141. In this case, the heater 121B having the blade-like shape is inserted into the substrate 151 of the stick substrate 150 and heats the substrate 151 of the stick substrate 150 from its inside. In another example, the heater 121B may be disposed so that the heater 121B covers the bottom 143 of the holder 140. In still another example, the heater 121B may be implemented as a combination of two or more selected from a first heater that covers the outer circumference of the holder 140, a second heater having the blade-like shape, and a third heater that covers the bottom 143 of the holder 140.

In another example, the holder 140 may include an opening/closing mechanism that at least partially opens and closes an outer shell defining the internal space 141. Examples of the opening/closing mechanism include a hinge. In addition, the holder 140 may sandwich the stick substrate 150 inserted into the internal space 141 by opening and closing the outer shell. In this case, the heater 121B may be at the sandwiching position of the holder 140 and may produce heat while pressing the stick substrate 150.

In addition, means for atomizing the aerosol source is not limited to heating by the heater 121B. For example, the means for atomizing the aerosol source may be induction heating.

In addition, the inhaler device 100B may also include the heater 121A, the liquid guide 122, the liquid storage 123, and the airflow path 180 according to the first configuration example. The air outlet hole 182 of the airflow path 180 may also serve as the air inlet hole to the internal space 141. In this case, a mixture fluid of the air and an aerosol generated by the heater 121A flows into the internal space 141, mixes further with an aerosol generated by the heater 121B, and then reaches the oral cavity of the user

### «2. Technical features»

### <2.1. System configuration example>

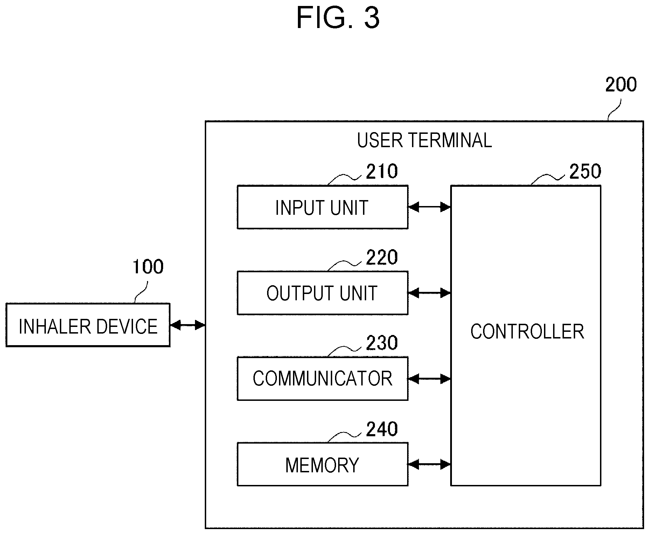

Fig. 3 is a diagram illustrating an example of the configuration of a system 1 according to the present embodiment. As illustrated in Fig. 3, the system 1 includes an inhaler device 100 and a user terminal 200.

### (1) Configuration of inhaler device 100

The inhaler device 100 may have any configuration example among the first configuration example and the second configuration example described above. In the following, inhalation of the aerosol generated by the inhaler device 100 by the user is simply referred to also as "inhalation" or "puff".

The inhaler device 100 according to the present embodiment uses a substrate to generate an aerosol to be inhaled by the user. A heater 121 is an example of a generator that generates an aerosol. The cartridge 120 and the flavor imparting cartridge 130 in the first configuration example, and the stick substrate 150 in the second configuration example are examples of a substrate according to the present invention. The inhaler device 100 generates an aerosol by using a substrate attached to the inhaler device 100. In the first configuration example, the cartridge 120 and the flavor imparting cartridge 130 connected to the power supply unit 110 are examples of the substrate attached to the inhaler device 100. In the second configuration example, the stick substrate 150 inserted into the inhaler device 100 is an example of the substrate attached to the inhaler device 100.

### (2) Configuration of user terminal 200

The user terminal 200 is a terminal device used by the user of the inhaler device 100. The user terminal 200 is, for example, any information processing device such as a smartphone, a tablet terminal, or a wearable device. As illustrated in Fig. 3, the user terminal 200 includes an input unit 210, an output unit 220, a communicator 230, a memory 240, and a controller 250.

The input unit 210 has a function of receiving input of various items of information. The input unit 210 may include an input device that receives input of information from the user. Examples of the input device include a button, a keyboard, a touch panel, and a microphone. The input unit 210 may further include various sensors such as an image sensor.

The output unit 220 has a function of outputting information. The output unit 220 may include an output device that outputs information to the user. Examples of the output device include a display device that displays information, a light-emitting device that emits light, a vibration device that vibrates, and a sound output device that outputs sound. An example of the display device is a display. An example of the light-emitting device is a light emitting diode (LED). An example of the vibration device is an eccentric motor. An example of the sound output device is a speaker. The output unit 220 outputs information input from the controller 250 to notify the user of the information.

The communicator 230 is a communication interface for transmitting and receiving information between the user terminal 200 and other devices. The communicator 230 performs communication in conformity with any wired or wireless communication standard. Such a communication standard may be, for example, a wireless local area network (LAN), a wired LAN, Wi-Fi (registered trademark), or Bluetooth (registered trademark).

The memory 240 stores various items of information for operation of the user terminal 200. The memory 240 may be a non-volatile storage medium such as flash memory.

The controller 250 functions as an arithmetic processing unit or a control circuit, and controls the overall operations of the user terminal 200 in accordance with various programs. The controller 250 includes an electronic circuit such as a central processing unit (CPU) or a microprocessor, for example. The controller 250 may further include a read only memory (ROM) that stores programs to be used, arithmetic parameters, and the like, and a random access memory (RAM) that temporarily stores parameters and the like that change as appropriate. The user terminal 200 executes various processes under the control of the controller 250. Processing of information input by the input unit 210, output of information by the output unit 220, transmission and reception of information by the communicator 230, and storage and reading of information by the memory 240 are examples of the processes controlled by the controller 250. Other processes executed by the user terminal 200, such as input of information to each structural element and processes based on information output from each structural element, are also controlled by the controller 250.

The functions of the controller 250 may be implemented by using an application. The application may be pre-installed or may be downloaded. Alternatively, the functions of the controller 250 may be implemented by progressive web apps (PWA).

### <2.2. Representation of heating profile>

### (1) Heating profile

The inhaler device 100 according to the present embodiment operates in accordance with a heating profile (corresponding to a first profile). The heating profile is information that defines an operation of generating an aerosol (i.e., an operation of heating the substrate), which is performed by the inhaler device 100. The inhaler device 100 heats the substrate in accordance with the heating profile to generate an aerosol.

More specifically, the heating profile is information indicating a time-series change of a parameter related to an operation of generating an aerosol, which is performed by the inhaler device 100. An example of the parameter is the temperature of the heater 121. A controller 116 controls the temperature of the heater 121 so that the heater 121 can achieve a temperature similar to a target temperature, where the target temperature is a temperature defined in the heating profile. The control of the temperature of the heater 121 can be implemented by known feedback control, for example. Specifically, the controller 116 causes electric power from a power supply 111 to be supplied to the heater 121 in the form of pulses based on pulse width modulation (PWM) or pulse frequency modulation (PFM). In this case, the controller 116 can adjust the duty ratio of electric power pulses to control the temperature of the heater 121.

In the feedback control, it is desirable that the controller 116 control electric power to be supplied to the heater 121, for example, the duty ratio described above, based on a difference between the temperature of the heater 121 and the target temperature or the like. The feedback control may be, for example, proportional-integral-differential controller (PID controller). The temperature of the heater 121 can be determined by, for example, measuring or estimating an electrical resistance value of a heating resistor of the heater 121. This is because the electrical resistance value of the heating resistor changes with temperature. The electrical resistance value of the heating resistor can be estimated by, for example, measuring an amount of voltage drop across the heating resistor. The amount of voltage drop across the heating resistor can be measured by a voltage sensor that measures a potential difference to be applied to the heating resistor. In another example, the temperature of the heater 121 may be measured by a temperature sensor installed near the heater 121.

In the first configuration example, heating is performed by the heater 121A at a time when a puff is detected. That is, the heater 121A performs heating each time a puff is detected. After the substrate is attached to the inhaler device 100A according to the first configuration example, the amount of the aerosol source contained in the substrate decreases each time a puff is taken. Eventually, the aerosol source is exhausted. Thus, the user typically replaces the substrate at a time when the aerosol source is exhausted.

In the second configuration example, by contrast, heating is started by the heater 121B at a time when the execution of an operation of giving an instruction to start heating is detected. During heating performed by the heater 121B, an aerosol is generated from the substrate. After the start of heating, the amount of the aerosol source contained in the substrate decreases with time. The heating by the heater 121B is stopped at a time when the aerosol source is exhausted. Thus, the user typically takes a puff during heating performed by the heater 121B.

A period during which a sufficient amount of aerosol is assumed to be generated is also referred to as a puffable period. By contrast, a period from the start of heating to the start of the puffable period is also referred to as a preheating period. The heating performed in the preheating period is also referred to as preheating. The user may be notified of the time at which the puffable period starts and the time at which the puffable period ends. In this case, the user can take a puff in the puffable period while referring to the notification.

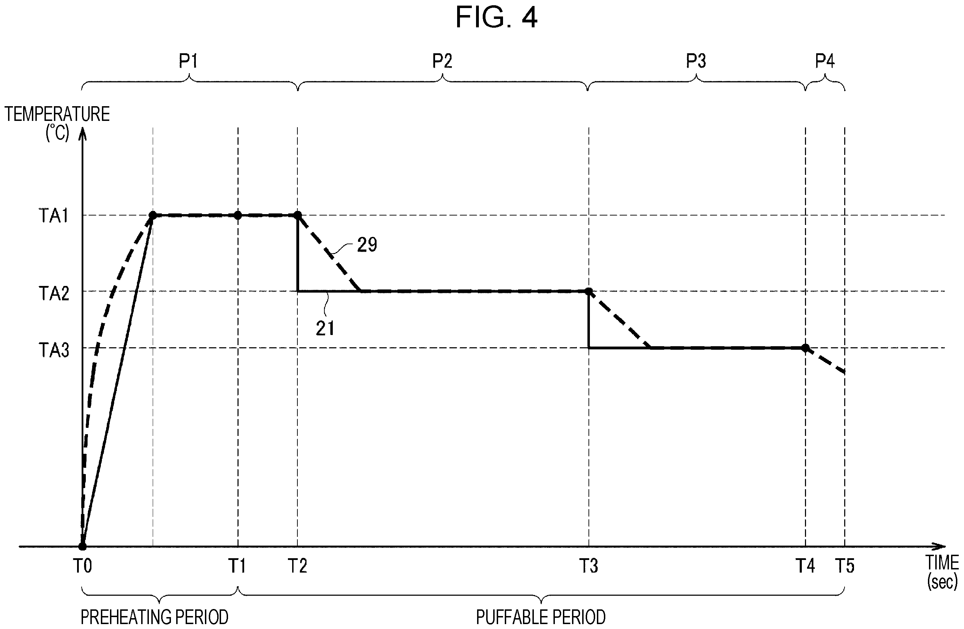

An example of a heating profile in the second configuration example will be described with reference to Fig. 4. Fig. 4 is a graph illustrating an example of a heating profile according to the present embodiment. In the graph, the horizontal axis represents time. In the graph, the vertical axis represents the temperature of the heater 121. In the graph, a solid line 21 indicates a time-series change of a target temperature in the heating profile. In the graph, a broken line 29 indicates a portion of a time-series change of an actual temperature of the heater 121 when the heater 121 operates in accordance with the heating profile, the portion having a large deviation from the heating profile.

Referring to the solid line 21 in the graph, a first target temperature TA1 is set during a first period P1 from a time T0 to a time T2. A second target temperature TA2 is set during a second period P2 from the time T2 to a time T3. A third target temperature TA3 is set during a third period P3 from the time T3 to a time T4. Accordingly, the controller 116 controls the temperature of the heater 121 toward the set target temperature in each of these periods. As a result, as indicated by the broken line 29 in the graph, the temperature of the heater 121 changes so as to follow the heating profile although a portion of the change has a deviation from the heating profile.

By a time T1, the substrate is sufficiently heated and a sufficient amount of aerosol is generated. Thus, the preheating period ends at the time T1, and the puffable period starts from the time T1. No target temperature is set in a fourth period P4 after the time T4. Thus, the controller 116 controls the heater 121 to stop heating. However, an aerosol is generated during a period in which residual heat remains in the heater 121 and the substrate. Thus, the puffable period ends at a time T5 after the time T4.

### (2) Superimposed display of heating profile and puff detection time

The user terminal 200 generates a display image that displays a heating profile from a start time point to an end time point and information related to a puff detection time in association with each other. The puff detection time is a time at which the user's inhalation of the aerosol generated by the inhaler device 100 is detected. An example of the start time point of the heating profile in the first configuration example is a time of attachment of a new substrate. An example of the end time point of the heating profile in the first configuration example is a time of removal of the attached substrate. An example of the start time point of the heating profile in the second configuration example is a time at which preheating is started and a time at which an inhalable period starts. An example of the end time point of the heating profile in the second configuration example is a time at which heating by the heater 121 ends and a time at which the inhalable period ends.

For example, the user terminal 200 receives, from the inhaler device 100, a heating profile to be used by the inhaler device 100 to generate an aerosol. The user terminal 200 further receives, from the inhaler device 100, information indicating that a puff is detected by a sensor 112. The inhaler device 100 may transmit information indicating that a puff is detected, immediately after the detection of the puff, or may collectively transmit pieces of information after the detection of puffs. The user terminal 200 generates, based on the pieces of information, a display image that displays the heating profile and puff detection times in association with each other, and outputs the generated display image. The user visually recognizes the display image and thus can recognize the relationship between the heating profile and the puff detection times. Furthermore, the user can recognize the relationship between the heating profile and the puff detection times while referring to the feeling of inhaling the aerosol.

The display image may include information indicating the positions of the puff detection times on the time axis of the heating profile. This configuration allows the user to more clearly recognize the relationship between the heating profile and the puff detection times.

The display image may include information indicating values of the parameter at the puff detection times. This configuration allows the user to recognize the relationship between the feeling of inhaling the aerosol and the parameters of the heating profile.

An example of the display image will be described hereinafter.

### - First display example

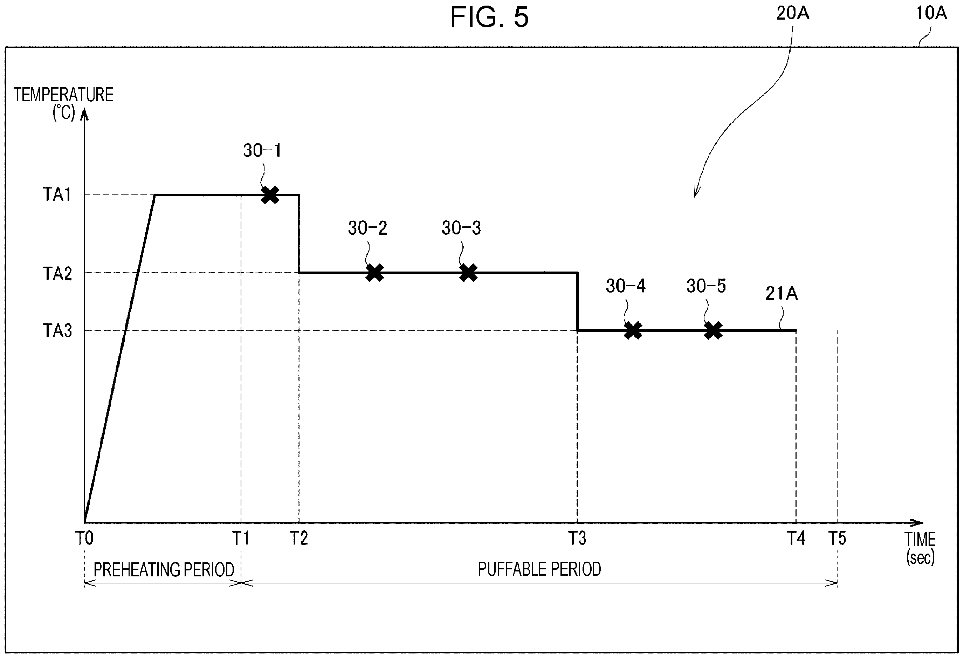

The display image may display the heating profile from the start time point to the end time point in association with information indicating a value of the parameter at a puff detection time detected from the start time point to the end time point. For example, in the second configuration example, the display image may display a heating profile in the period from the start of preheating to the end of the puffable period in association with a value of the parameter at a puff detection time detected in this period. An example of the display image will be described with reference to Fig. 5.

Fig. 5 is a diagram illustrating an example of the display image generated by the user terminal 200 according to the present embodiment. A display image 10A illustrated in Fig. 5 includes a graph 20A depicting a line 21A indicating a heating profile from the start of preheating to the end of the puffable period. In the graph 20A, the horizontal axis represents time. In the graph 20A, the vertical axis represents a parameter of the heating profile (i.e., the target temperature of the heater 121). In the graph 20A, X-shaped points 30 (30-1 to 30-5) indicate values of the parameter at puff detection times for puffs detected in the period from the start of preheating to the end of the puffable period. The values of the points 30 on the horizontal axis correspond to the puff detection times. The values of the points 30 on the vertical axis correspond to values of the parameter at the puff detection times.

The display image according to the first display example is displayed after the puffable period ends, that is, after the user finishes taking a series of puffs. Accordingly, after finishing taking a series of puffs, the user can collectively recognize the relationship between the heating profile for the series of puffs and the puff detection times.

### - Second display example

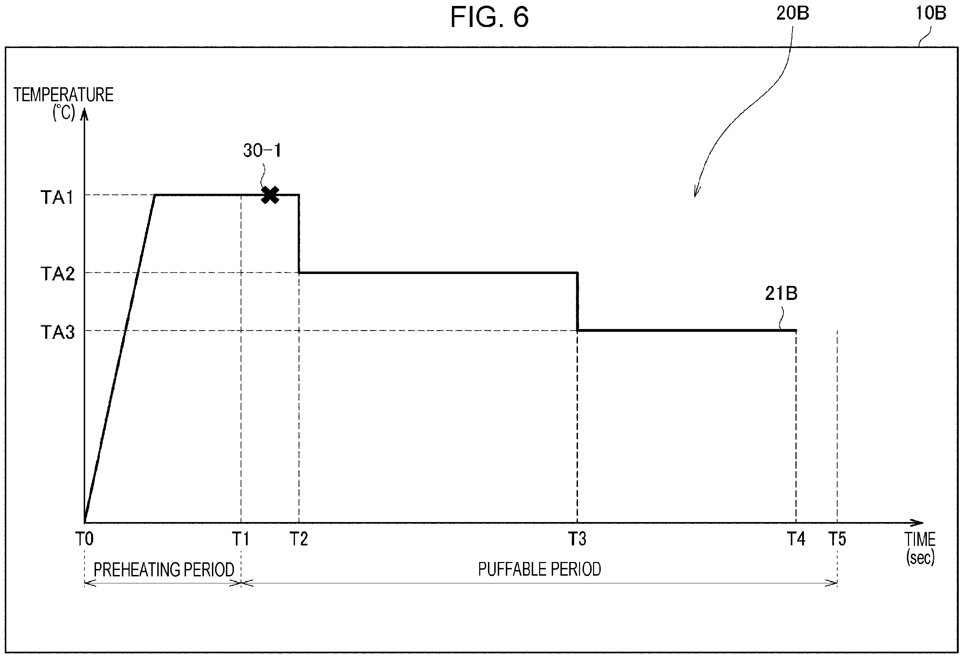

The display image may display the heating profile from the start time point to the end time point in association with information indicating a value of the parameter at a puff detection time detected from the start time point to the current time point. For example, in the second configuration example, the display image may display a heating profile from the start of preheating to the end of the puffable period in association with a value of the parameter at a puff detection time detected up to the current time point. An example of the display image will be described with reference to Fig. 6.

Fig. 6 is a diagram illustrating an example of the display image generated by the user terminal 200 according to the present embodiment. A display image 10B illustrated in Fig. 6 includes a graph 20B depicting a line 21B indicating a heating profile from the start of preheating to the end of the puffable period. In the graph 20B, the horizontal axis represents time. It is assumed that the current time is the time T2. In the graph 20B, the vertical axis represents the target temperature of the heater 121. In the graph 20B, an X-shaped point 30-1 indicates a value of the parameter at a puff detection time for a puff detected in the period from the start of preheating to the current time point. Each time a puff is taken by the user, a point 30 is added to the graph 20B.

The display image according to the second display example is displayed in real time during heating performed by the heater 121. This allows the user to recognize the relationship between the heating profile and a puff detection time in real time while performing a puff action.

### - Third display example

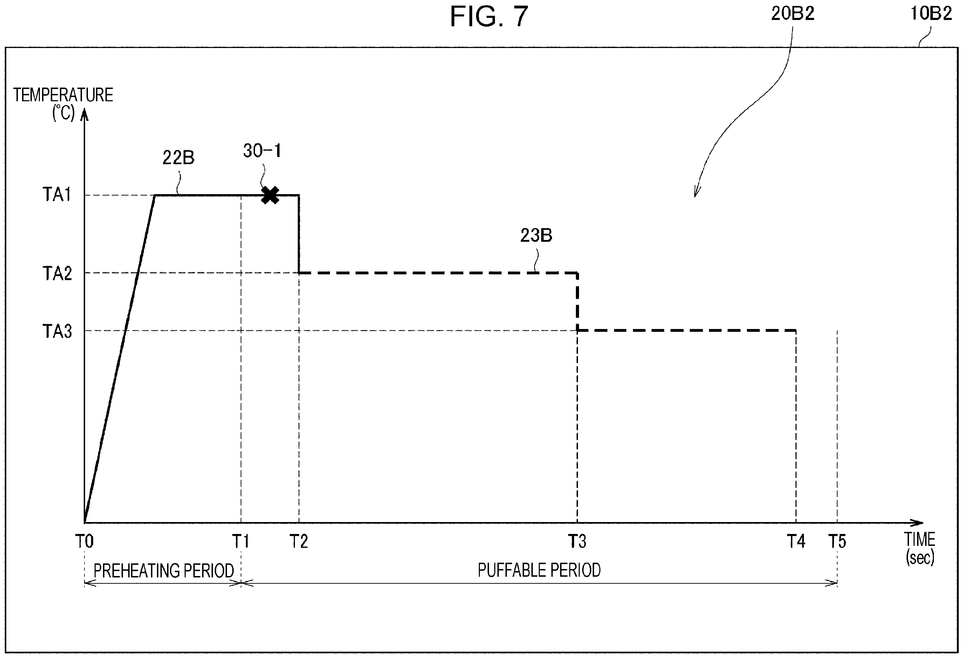

The display image may display an entire time section from the start time point to the end time point, based on an elapsed time from the start of preheating, such that a section that has already elapsed is displayed in a first display mode and a section that has not yet elapsed is displayed in a second display mode. For example, the user terminal 200 receives, from the inhaler device 100, information indicating the start of preheating, and starts measurement of the elapsed time from the start of preheating in response to receipt of the information indicating the start of preheating. Then, the user terminal 200 displays the entire time section from the start time point to the end time point, based on the measured elapsed time, such that a section that has already elapsed is displayed in the first display mode and a section that has not yet elapsed is displayed in the second display mode. An example of the display image will be described with reference to Fig. 7.

Fig. 7 is a diagram illustrating an example of the display image generated by the user terminal 200 according to the present embodiment. A display image 10B2 illustrated in Fig. 7 includes a line 22B indicating a heating profile from the start of preheating to a section that has already elapsed, and a line 23B indicating a heating profile of a section that has not yet elapsed. In a graph 20B2, the horizontal axis represents time. It is assumed that the current time is the time T2. In the graph 20B2, the vertical axis represents the target temperature of the heater 121. In the graph 20B2, an X-shaped point 30-1 indicates a value of the parameter at a puff detection time for a puff detected in the period from the start of preheating to the current time point. Each time a puff is taken by the user, a point 30 is added to the graph 20B2.

In the display screen 10B2, the line 22B indicating the heating profile from the start of preheating to the section that has already elapsed is displayed in the first display mode. In the display screen 10B2, the first display mode is a solid line. In the display screen 10B2, by contrast, the line 23B indicating the heating profile of the section that has not yet elapsed is displayed in the second display mode. In the display screen 10B2, the first display mode is a broken line.

The first display mode and the second display mode are different display modes. The first display mode and the second display mode may be any modes as long as these display modes are different from each other, such as different colors, different shades, different thicknesses, or different shapes. For example, the lines 22B and the lines 23B may be displayed in different colors, such as red and black, respectively, in the display screen 10B2. That is, for example, the heating profile in the section that has already elapsed since the start of preheating is displayed in red in the display screen 10B2, and the heating profile in the section that has not yet elapsed since the start of preheating is displayed in black in the display screen 10B2.

The display image according to the third display example is displayed in real time during heating performed by the heater 121. This allows the user to recognize the relationship between the heating profile and a puff detection time in real time while performing a puff action.

### - Fourth display example

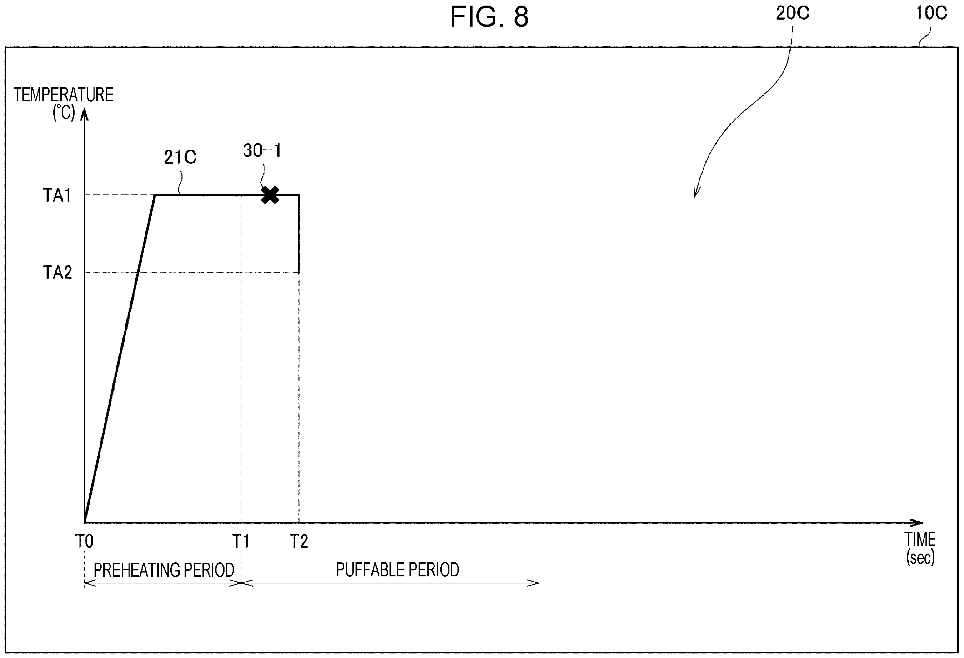

The display image may display the heating profile from the start time point to the current time point in association with information indicating a value of the parameter at a puff detection time detected from the start time point to the current time point. For example, in the second configuration example, the display image may display a heating profile in a period from the start of preheating to the current time point in association with a value of the parameter at a puff detection time detected in this period. An example of the display image will be described with reference to Fig. 8.

Fig. 8 is a diagram illustrating an example of the display image generated by the user terminal 200 according to the present embodiment. A display image 10C illustrated in Fig. 8 includes a graph 20C depicting a line 21C indicating a heating profile from the start of preheating to the current time point. In the graph 20C, the horizontal axis represents time. It is assumed that the current time is the time T2. In the graph 20C, the vertical axis represents the target temperature of the heater 121. In the graph 20C, an X-shaped point 30-1 indicates a value of the parameter at a puff detection time for a puff detected from the start of preheating to the current time point. As time passes, the line 21C indicating the heating profile is extended. Each time a puff is taken by the user, a point 30 is added to the graph 20C.

The display image according to the fourth display example is displayed in real time during heating performed by the heater 121. This allows the user to recognize the relationship between the heating profile and a puff detection time in real time while performing a puff action.

### - Fifth display example

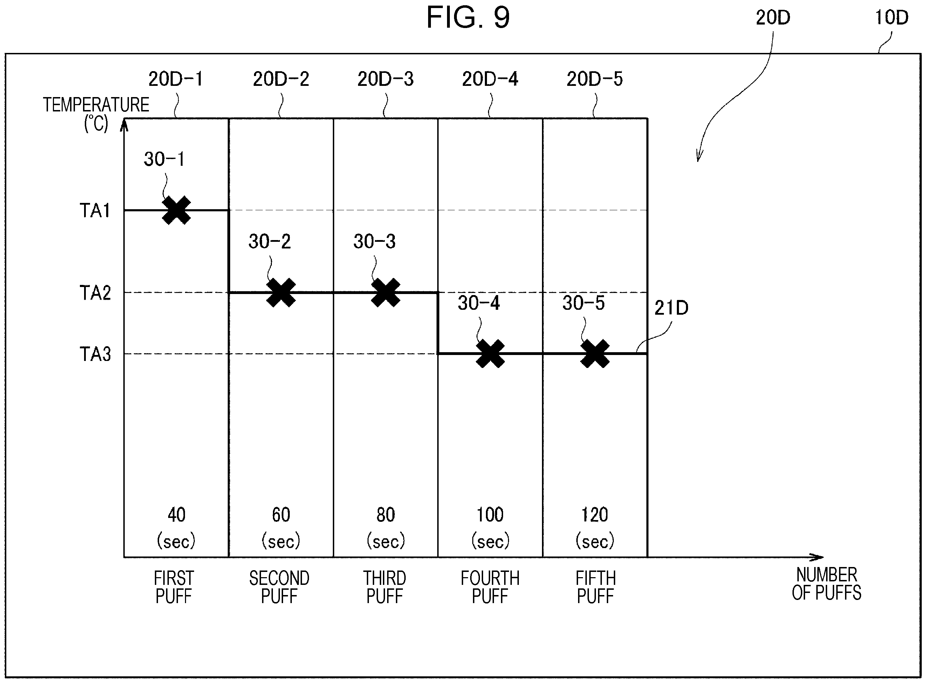

In the display image, a heating profile in part of the entire time section from the start time point to the end time point, the part including a puff detection time, may be extracted and displayed. For example, in the second configuration example, the display image may display an extracted heating profile including a puff detection time in the period from the start of preheating to the end of the puffable period in association with information indicating a value of the parameter at the puff detection time. An example of the display image will be described with reference to Fig. 9.

Fig. 9 is a diagram illustrating an example of the display image generated by the user terminal 200 according to the present embodiment. A display image 10D illustrated in Fig. 9 includes a graph 20D (20D-1 to 20D-5) obtained by extracting a line 21D indicating a heating profile including puff detection times in the period from the start of preheating to the end of the puffable period. Each of graphs 20D-1 to 20D-5 depicts a heating profile at a time when a puff is detected. In the graph 20D, the horizontal axis represents the number of puffs (the cumulative number of puffs taken). In each of the graphs 20D-1 to 20D-5, the horizontal axis represents time. In each of the graphs 20D-1 to 20D-5, the vertical axis represents the target temperature of the heater 121. In each of the graphs 20D-1 to 20D-5, an elapsed time from the start of preheating and the number of puffs detected are displayed in association with each other. The elapsed time from the start of preheating may not be displayed. In addition to or instead of the elapsed time from the start of preheating, an elapsed time from the time at which the first puff is detected may be displayed. In the graph 20D, X-shaped points 30 (30-1 to 30-5) indicate values of the parameter at the puff detection times.

The fifth display example allows the user to recognize the relationship between the heating profile and the puff detection times with a focus on the puff detection times.

### - Sixth display example

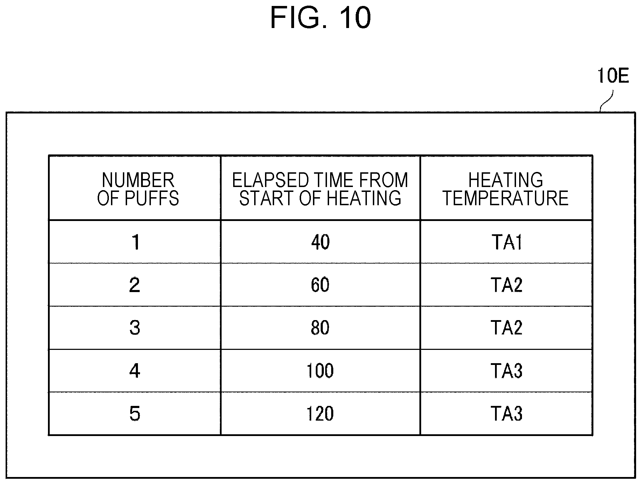

In the display images according to the first to fifth display examples, a heating profile is displayed as a graph. A value of the parameter at a puff detection time is displayed in association with the heating profile displayed as the graph. However, the present invention is not limited to such examples. The display image may display information related to puff detection times as a table. An example of the display image will be described with reference to Fig. 10.

Fig. 10 is a diagram illustrating an example of the display image generated by the user terminal 200 according to the present embodiment. A display image 10E illustrated in Fig. 10 includes a table indicating the number of puffs, an elapsed time from the start of heating (i.e., the start of preheating), and a heating temperature (i.e., a temperature of the heater 121) at a puff detection time detected in the period from the start of preheating to the end of the puffable period. The elapsed time from the start of heating may not be displayed. In the display image, in addition to or instead of the elapsed time from the start of preheating, an elapsed time from the time at which the first puff is detected may be displayed.

The sixth display example allows the user to recognize the relationship between the heating profile and the puff detection times with a focus on the puff detection times.

### - Seventh display example

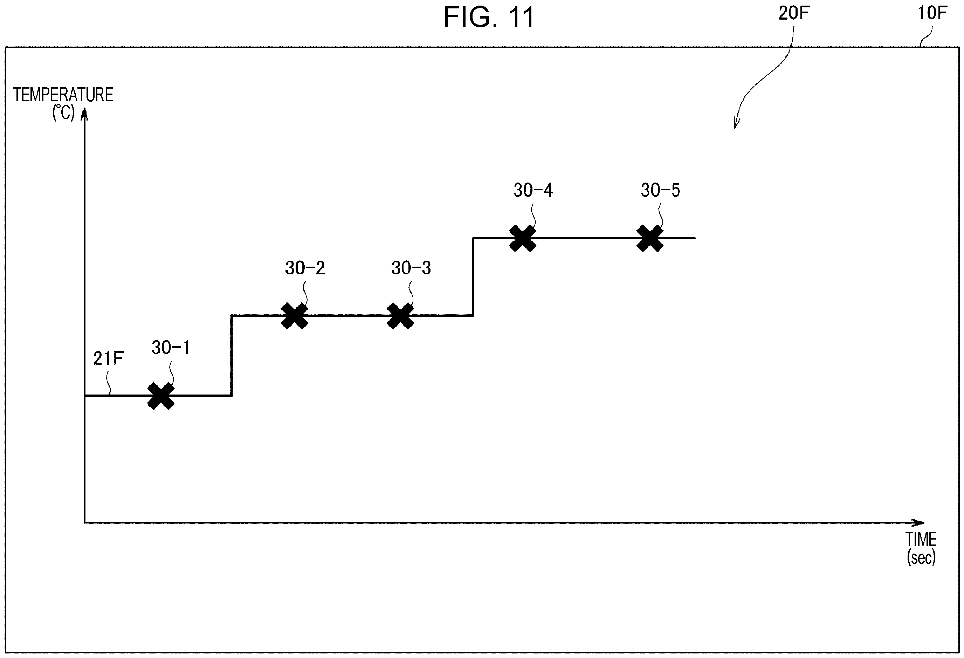

The display examples described above illustrate examples of a display image related to the inhaler device 100 according to the second configuration example. A similar display image is also generated for the inhaler device 100 according to the first configuration example. An example of the display image related to the inhaler device 100 according to the first configuration example will be described with reference to Fig. 11.

Fig. 11 is a diagram illustrating an example of the display image generated by the user terminal 200 according to the present embodiment. A display image 10F illustrated in Fig. 11 includes a graph 20F depicting a line 21F indicating a heating profile from attachment of a new substrate to removal of the new substrate. The line 21F indicates a time-series change of the target temperature for heating performed in response to detection of a puff. In the graph 20F, the horizontal axis represents time. The illustrated heating profile starts at a time point when the first puff is detected. That is, in the illustrated graph, the horizontal axis represents the elapsed time from the time at which the first puff is detected. In the graph 20F, the vertical axis represents the target temperature of the heater 121. In the graph 20F, X-shaped points 30 (30-1 to 30-5) correspond to values of the parameter at puff detection times. The values of the points 30 on the horizontal axis correspond to the puff detection times. The values of the points 30 on the vertical axis correspond to target temperatures at the puff detection times. In the graph 20F, the horizontal axis may represent the number of puffs instead of time.

As in the first display example, the display image 10F illustrated in Fig. 11 displays a heating profile from the start time point to the end time point in association with information indicating values of the parameter at puff detection times detected in this period. The display image related to the inhaler device 100 according to the first configuration example may be displayed in a manner similar to that of the display images according to the second to sixth display examples in addition to the first display example.

### <2.3. Customization of heating profile>

The user terminal 200 generates a display image that displays a heating profile. Then, the user terminal 200 changes the heating profile and the display of the heating profile in the display image in accordance with a user operation on the parameter to be operated in the display image. The parameter to be operated is a parameter that can be changed in accordance with a user operation among parameters of the heating profile. This configuration allows the user to change the heating profile while visually recognizing the change of the heating profile. Accordingly, usability in customizing the heating profile is improved.

Specifically, the user terminal 200 changes the parameter to be operated for the heating profile in accordance with a user operation of changing the parameter to be operated. Then, the user terminal 200 updates the parameter to be operated for the heating profile displayed in the display image to the changed parameter. This configuration allows the user to intuitively change the parameter for the heating profile.

As described above, the display image may display the heating profile in association with information indicating puff detection times. In this case, the user can customize the heating profile while recognizing the relationship between the heating profile and the puff detection times while referring to the feeling of inhaling the aerosol.

The user terminal 200 controls the inhaler device 100 so that the inhaler device 100 can operate in accordance with the heating profile on which the change has been made. For example, the user terminal 200 receives and displays a profile on which the change has not been made from the inhaler device 100, and transmits a heating profile on which the change has been made by the user to the inhaler device 100. At this time, the user terminal 200 may transmit the heating profile on which the change has been made or may transmit the difference between the heating profile on which the change has not been made and the heating profile on which the change has been made. The next time an aerosol is generated, the inhaler device 100 operates in accordance with the heating profile on which the change has been made. This configuration allows the user to be free to customize the operation of the inhaler device 100. This allows the user to, for example, search for a heating profile that achieves desired smoking quality, with repeated customizations.

### (1) Basic display image and customization

The following describes an example of customization of the display image according to the first display example described above.

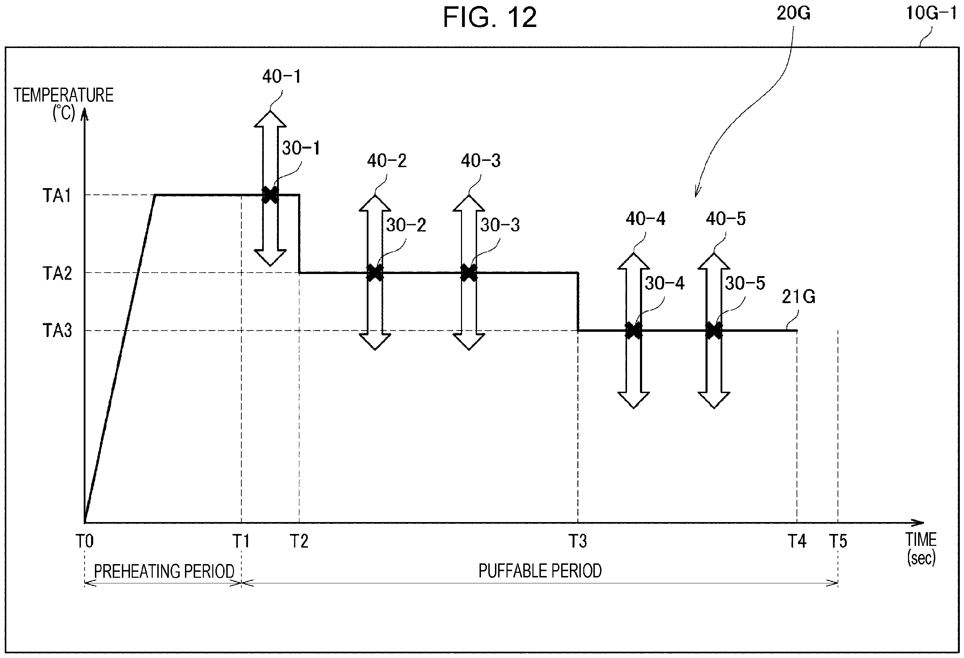

Fig. 12 is a diagram illustrating an example of the display image generated by the user terminal 200 according to the present embodiment. A display image 10G-1 illustrated in Fig. 12 includes a graph 20G, a line 21G, and points 30, and such elements have features similar to those of the elements having the corresponding reference numerals illustrated in Fig. 5. That is, the display image 10G-1 displays the line 21G indicating a heating profile from the start time point to the end time point in association with the points 30 indicating values of the parameter at puff detection times detected from the start time point to the end time point.

The parameter to be operated may be a value of the parameter at a puff detection time. For example, the user performs an operation of selecting one of the points 30 indicating the values of the parameter at the puff detection times and shifting the selected point 30 up and down. The user terminal 200 changes the value of the parameter corresponding to the selected point 30 in accordance with the user operation. This configuration allows the user to customize the heating profile while referring to the feeling of inhaling the aerosol.

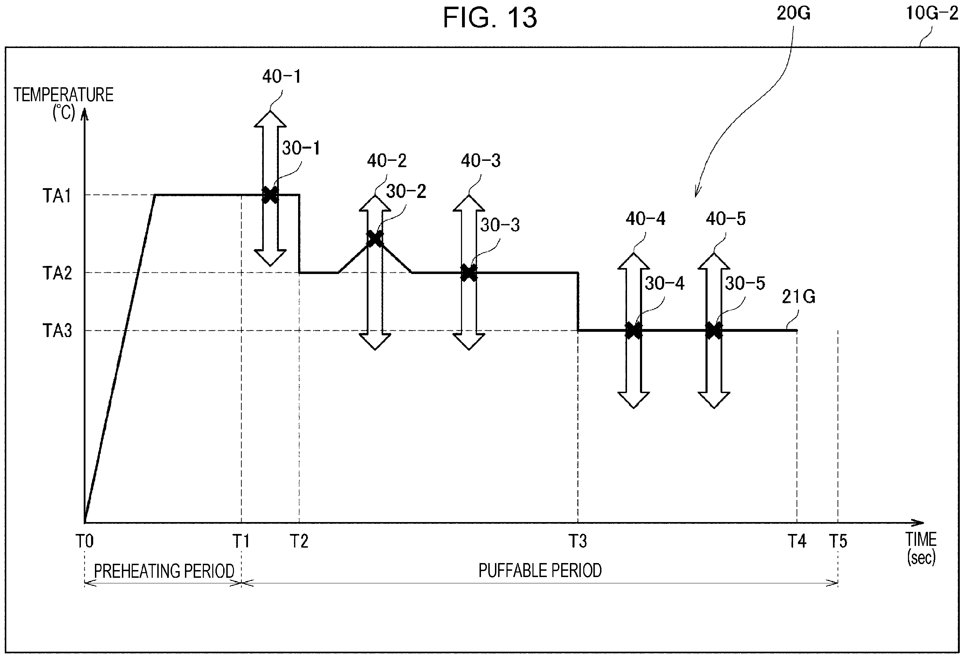

In an example, in the display image 10G-1 illustrated in Fig. 12, a point 30-2 is selected and an operation of increasing the parameter is performed to produce a changed display image 10G-2, which is illustrated in Fig. 13. Fig. 13 is a diagram illustrating an example of the display image generated by the user terminal 200 according to the present embodiment. As illustrated in Fig. 13, the value of the parameter at the point 30-2 is increased from the state illustrated in Fig. 12. In addition, the shape of a portion of the line 21G at and around the point 30-2 is changed from a straight-line shape in Fig. 12 to an inverted V shape with the point 30-2 as a peak in Fig. 13. In this way, the user terminal 200 may also change a value of the parameter at another time that is continuous, in the time axis direction, with the time at which the value of the parameter is changed by the user operation.

The display image 10G-1 may include information indicating that the parameter to be operated is changeable. Arrows 40 (40-1 to 40-5) are examples of such information. For example, in the example illustrated in Fig. 12, the arrows 40 are superimposed on the points 30. This configuration allows the user to easily recognize that the values of the parameter at the points 30 is changeable.

The display image 10G-1 may include information indicating a range in which the parameter to be operated is changeable. The ranges of the arrows 40 in the vertical axis direction are examples of such information. For example, in the example illustrated in Fig. 12, each of the values of the parameter at the points 30 can be changed up and down in the range indicated by a corresponding one of the superimposed arrows 40. This configuration allows the user to easily recognize a range in which the parameter is changeable.

The range in which the parameter to be operated is changeable is determined based on the performance of the heater 121, settings made by the user, and the like. The ranges in which the plurality of parameters to be operated are changeable may be different or the same.

The display image in the first display example is displayed after the puffable period ends, that is, after the user finishes taking a series of puffs. Accordingly, the user can customize the heating profile while collectively recognizing the relationship between the heating profile for the series of puffs and the puff detection times.

### (2) Variations of display image and customization

### - First variation

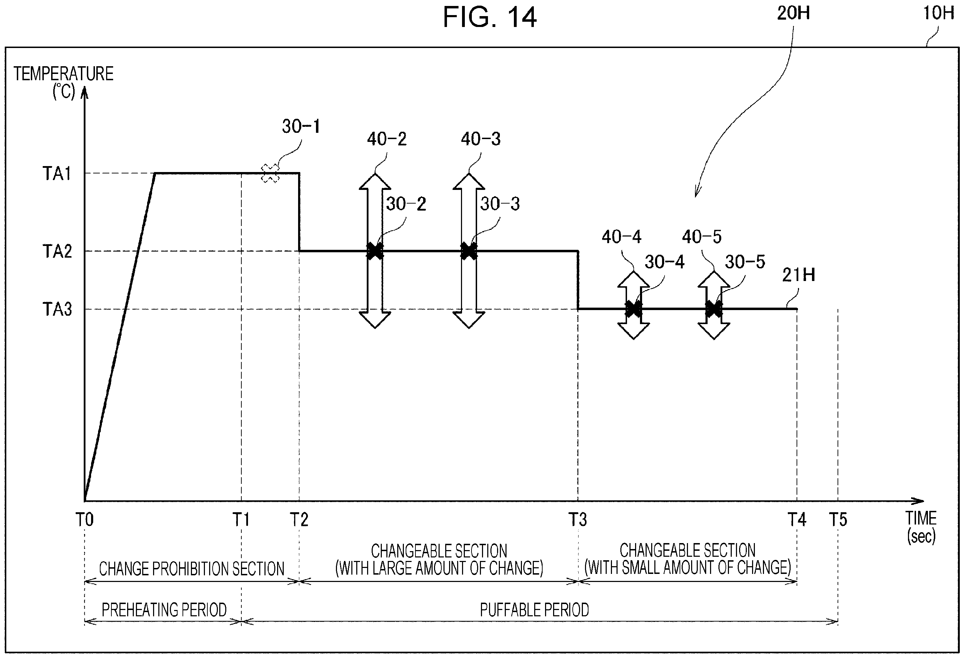

The heating profile may be divided into a plurality of time sections, and whether the parameter is changeable and the range in which the parameter is changeable may be set for each time section. This configuration will be described with reference to Fig. 14.

Fig. 14 is a diagram illustrating an example of the display image generated by the user terminal 200 according to the present embodiment. A display image 10H illustrated in Fig. 14 includes a graph 20H, a line 21H, points 30, and arrows 40, and such elements have features similar to those of the elements having the corresponding reference numerals illustrated in Fig. 12.

The section from the time T0 to the time T2 is a change prohibition section in which the parameter is prohibited from being changed. Thus, an operation for changing the parameter for the point 30-1 is not accepted. To indicate non-acceptance of the change, the point 30-1 is represented by a broken line. Since no change is allowed for the parameter, none of the arrows 40 is superimposed on the point 30-1. In the section in which the parameter is prohibited from being changed, no point 30 may be initially displayed.

The section from the time T2 to the time T3 is a changeable section in which the parameter is changeable, and is a section in which a large amount of change is allowed. Thus, relatively long arrows 40-2 and 40-3 are superimposed on the point 30-2 and the point 30-3, respectively, to indicate a large changeable range.

The section from the time T3 to the time T4 is a changeable section in which the parameter is changeable, and is a section in which a small amount of change is allowed. Thus, relatively short arrows 40-4 and 40-5 are superimposed on the point 30-4 and the point 30-5, respectively, to indicate a small changeable range.

Whether the parameter is changeable and the range in which the parameter is changeable may be set for each time section in accordance with characteristics contributing to the generation of aerosol, such as the characteristics of the heater 121 and the characteristics of the substrate. This configuration makes it possible to customize the heating profile within a range in which an aerosol is appropriately generated.

### - Second variation

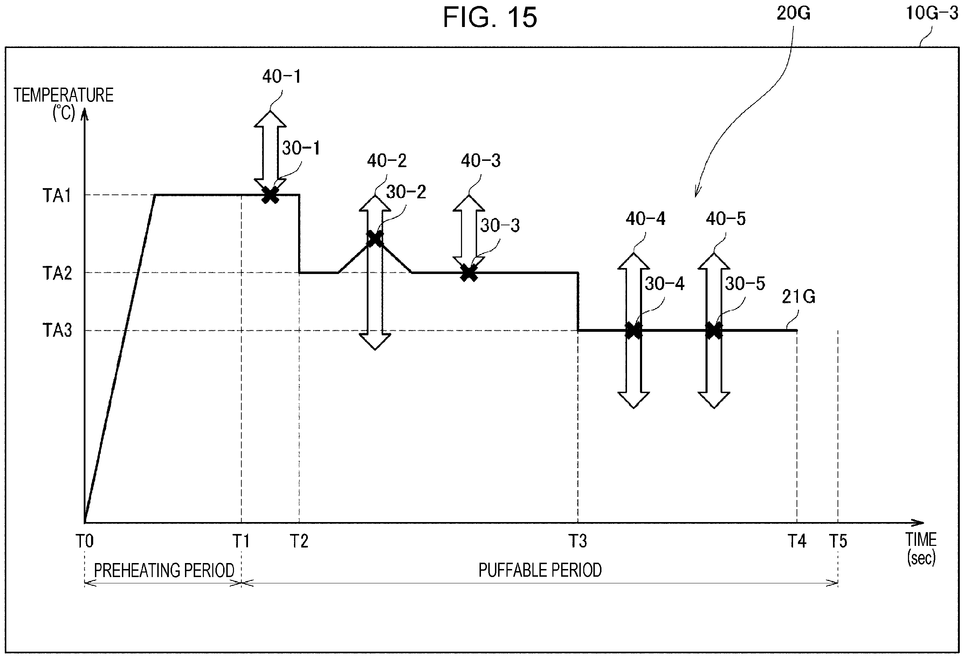

The user terminal 200 may change the range in which the parameter to be operated is changeable, in response to another parameter to be operated being changed in accordance with a user operation. In an example, in the display image 10G-1 illustrated in Fig. 12, the point 30-2 is selected and an operation of increasing the parameter is performed to produce a changed display image 10G-3, which is illustrated in Fig. 15.

Fig. 15 is a diagram illustrating an example of the display image generated by the user terminal 200 according to the present embodiment. A graph 20G illustrated in Fig. 15 has a shape similar to that of the graph 20G illustrated in Fig. 13. Further, the values of the parameter at the point 30-1 and the point 30-3, which are continuous with the point 30-2 in the time axis direction, are changeable in a range that is reduced such that the values of the parameter can only be increased. In this way, the range in which the values of the parameter at the point 30-1 and the point 30-3 are changeable is changed in accordance with the change of the value of the parameter at the point 30-2.

Such a change of the range in which the parameter to be operated is changeable, which is caused by the change of another parameter to be operated, may be set in accordance with various items of information contributing to the generation of aerosol, such as the characteristics of the heater 121, the characteristics of the substrate, and the interval between puffs. This configuration makes it possible to customize the heating profile within a range in which an aerosol is appropriately generated.

In an example, the range in which the parameter to be operated is changeable is initially set based on the characteristics of the heater 121 and the characteristics of the substrate. Then, the user terminal 200 sets a range in which another parameter to be operated is changeable, based on the value of the changed parameter to be operated and a time interval between the changed parameter to be operated and the other parameter to be operated. For example, it is assumed that the range in which the parameter to be operated is changeable is initially set from 200°C to 250°C. Further, it is assumed that the value of the parameter at a certain point 30 is changed from 230°C to 240°C by a user operation. In this case, the user terminal 200 sets the range in which the value of the parameter at another point 30 having a time interval of 10 seconds from the changed point 30 is changeable from 230°C to ±10 degrees. By contrast, the user terminal 200 sets the range in which the value of the parameter at another point 30 having a time interval of 20 seconds from the changed point 30 is changeable from 230°C to ±20 degrees.

### - Third variation

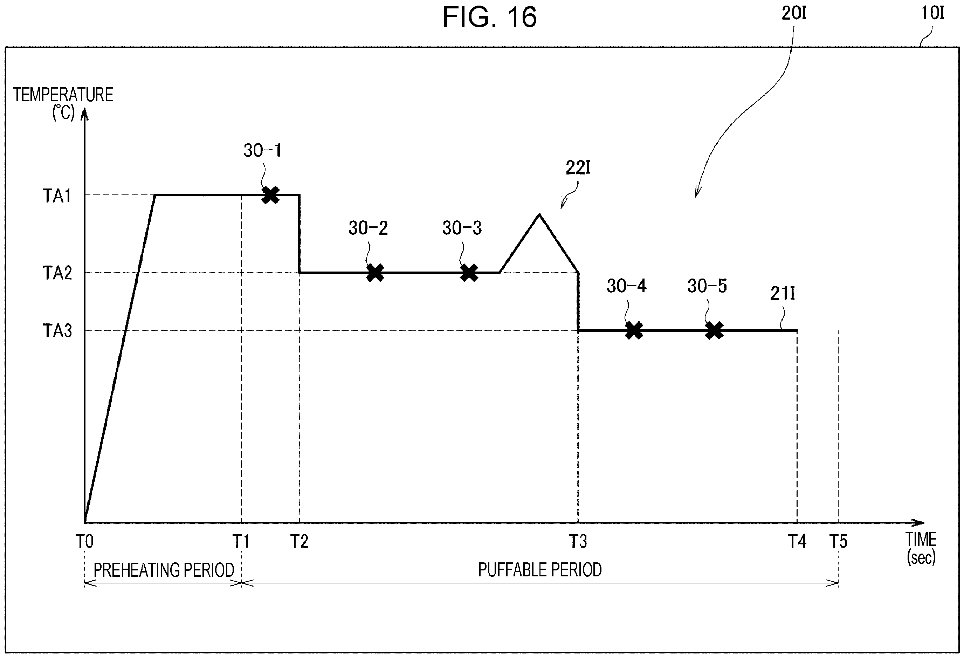

The parameter to be operated may include a value of the parameter at a time other than a puff detection time. This configuration will be described with reference to Fig. 16.

Fig. 16 is a diagram illustrating an example of the display image generated by the user terminal 200 according to the present embodiment. A display image 10I illustrated in Fig. 16 includes a graph 201, a line 21I, and points 30, and such elements have features similar to those of the elements having the corresponding reference numerals illustrated in Fig. 12. The user may directly change the line 21I as an operation of changing a value of the parameter at a time other than a puff detection time. Fig. 16 illustrates a heating profile in which a portion of the line 21I indicating the heating profile, which is represented by reference numeral 22I, has been selected and an operation of increasing the parameter has been performed. This configuration makes it possible to achieve more flexible customization.

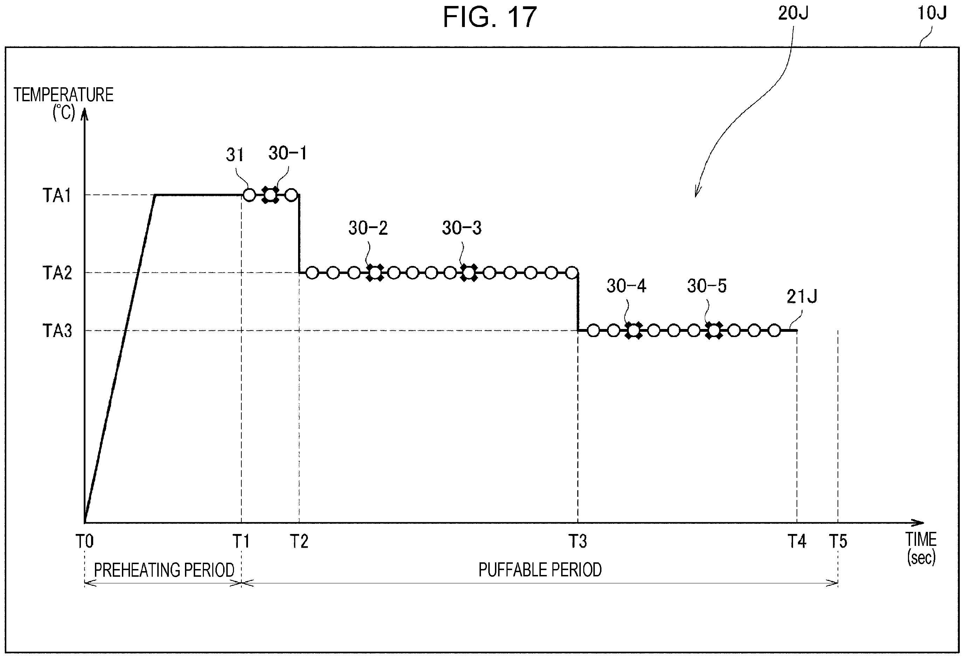

Here, the display image desirably displays the parameter to be operated in a mode different from that of a parameter that is not to be operated. The points 30 indicating values of the parameter to be operated, which are illustrated in Fig. 12 and the like, are examples of display in a mode different from that of a parameter that is not to be operated. It is also desirable that the parameter to be operated be displayed in a mode different from that of a parameter that is not to be operated even when the parameter to be operated is a value of the parameter at a time other than a puff detection time. This configuration will be described with reference to Fig. 17.

Fig. 17 is a diagram illustrating an example of the display image generated by the user terminal 200 according to the present embodiment. A display image 10J illustrated in Fig. 17 includes a graph 20J, a line 21J, and points 30, and such elements have features similar to those of the elements having the corresponding reference numerals illustrated in Fig. 12. In Fig. 17, a point 31 indicating a parameter to be operated is included in addition to the points 30 indicating values of the parameter at puff detection times. The user selects the point 31 and performs an operation of shifting the point 31 up and down. The user terminal 200 changes the value of the parameter corresponding to the selected point 31 in accordance with the user operation. This configuration makes it possible to improve visibility of a customizable parameter.

### - Fourth variation

The display image may further display another heating profile to be compared with the heating profile. This configuration allows the user to customize the heating profile while comparing it with another heating profile, making it possible to improve usability. The display image may display a plurality of heating profiles to be compared.

The heating profile to be compared may be a heating profile on which no change has been made. This configuration allows the user to customize the heating profile using a heating profile on which no change has been made as a clue.

Alternatively, the heating profile to be compared may be a previous heating profile. The previous heating profile is a heating profile that has been set in the inhaler device 100. For example, the previous heating profile is a heating profile that has been set in the inhaler device 100 at any time before the heating profile currently set in the inhaler device 100. Alternatively, the previous heating profile may be a heating profile previously customized by the user.

In this case, the inhaler device 100 or the user terminal 200 may store information related to a heating profile that has been set in the inhaler device 100 or a heating profile previously customized by the user. The user terminal 200 may display the previous heating profile in the display image, based on information related to the previous heating profile, which is stored in the inhaler device 100 or the user terminal 200. In a case where a plurality of previous heating profiles are present, the user terminal 200 may display, in the display image, a heating profile selected by the user from the plurality of previous heating profiles.

Alternatively, the user terminal 200 may display a plurality of previous heating profiles in the display screen as heating profiles to be compared. In this case, the plurality of previous heating profiles may be displayed in the display screen using different display modes. The respective display modes to be applied to the plurality of previous heating profiles may be any modes as long as these display modes are different from each other, such as different colors, different shades, different thicknesses, or different shapes. The plurality of heating profiles may be displayed in different colors such that, for example, a first previous heating profile is displayed with a red line and a second previous heating profile is displayed with a black line. The number of heating profiles to be displayed in the display screen may be selected as desired by the user. For example, in a case where five previous heating profiles are selected by the user, the five previous heating profiles may be displayed in the display screen.

This configuration allows the user to customize the heating profile using a previous heating profile as a clue. For example, in a case where a heating profile previously customized by the user is displayed in the display screen, the user can customize the heating profile to a heating profile desired by the user by trial and error, using a previously customized heating profile as a clue, while checking the difference from the previously customized heating profile. Specifically, the user can adjust the heating profile to be customized to a heating profile more desirable for the user while checking the difference from, for example, a first previous heating profile that is the most recently customized heating profile and a second previous heating profile that is the heating profile customized before the most recently customized heating profile.

As an alternative, the heating profile to be compared may be a standard heating profile. An example of the standard profile is a heating profile prepared in advance for each substrate. Other examples of the standard profile include heating profiles that are responsive to smoking characteristics, such as a heating profile for strong smoking characteristics, a heating profile for standard smoking characteristics, and a heating profile for mild smoking characteristics. The user terminal 200 may display, in the display image, a heating profile selected by the user from a plurality of standard heating profiles. This configuration allows the user to customize the heating profile using a standard heating profile as a clue.

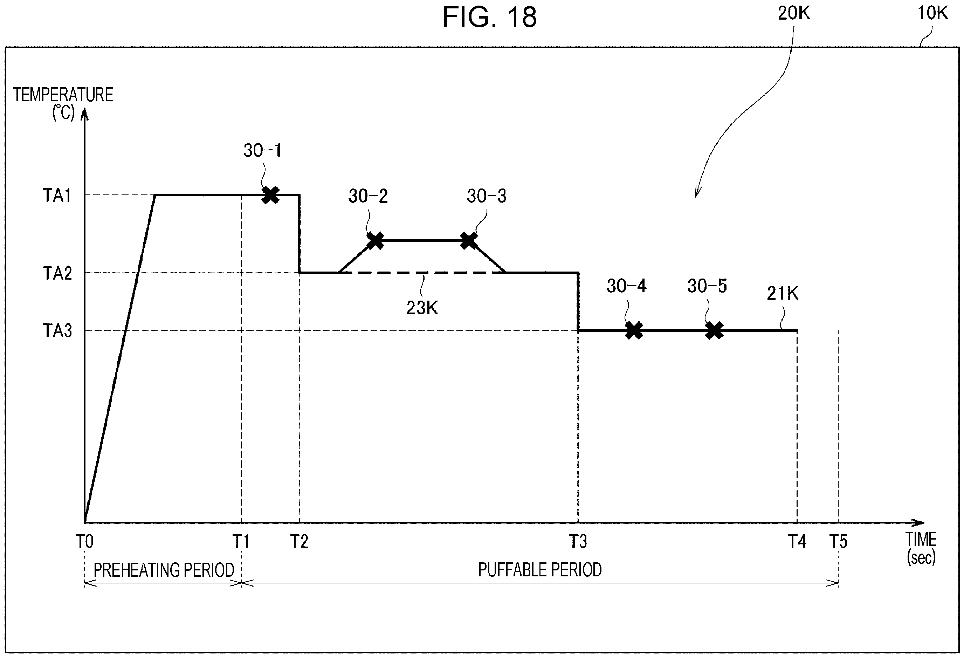

Fig. 18 is a diagram illustrating an example of the display image generated by the user terminal 200 according to the present embodiment. A display image 10K illustrated in Fig. 18 includes a graph 20K, a line 21K, and points 30, and such elements have features similar to those of the elements having the corresponding reference numerals illustrated in Fig. 12. In Fig. 18, a line 23K indicating a heating profile to be compared is displayed together with the line 21K indicating a heating profile on which a change has been made by the user. The line 21K and the line 23K are preferably displayed in different modes such that the line 21K and the line 23K are displayed by a solid line and a broken line or are displayed in different colors. This configuration allows the user to customize the heating profile while recognizing the difference between the line 23K and the line 21K. In Fig. 18, the line 23K indicating the heating profile to be compared is displayed only in a portion where a difference occurs from the heating profile. Alternatively, the line 23K may be displayed over the entire period from the time T0 to the time T4.

### (3) Other display examples

The foregoing has described an example of customization of the display image according to the first display example described above. The display images according to the second to seventh display examples may be customized. The following describes an example of customization of the display images according to the second to seventh display examples.

### - Second display example

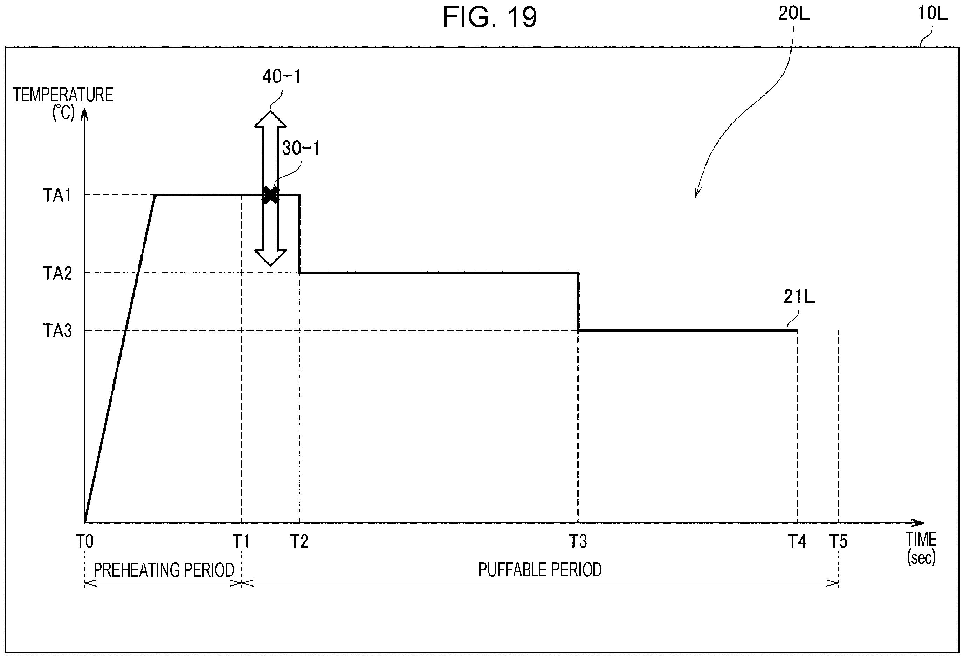

Fig. 19 is a diagram illustrating an example of the display image generated by the user terminal 200 according to the present embodiment. A display image 10L illustrated in Fig. 19 includes a graph 20L, a line 21L, and a point 30, and such elements have features similar to those of the elements having the corresponding reference numerals illustrated in Fig. 6. That is, the display image 10L displays the line 21L indicating a heating profile from the start time point to the end time point in association with a point 30-1 indicating a value of the parameter at a puff detection time detected from the start time point to the current time point. The user can change the value of the parameter at the point 30-1 within a changeable range indicated by an arrow 40-1.

In the display image according to the second display example, a value of the parameter at a puff detection time is displayed in real time. This allows the user to customize the heating profile in real time while performing a puff action.

### - Third display example

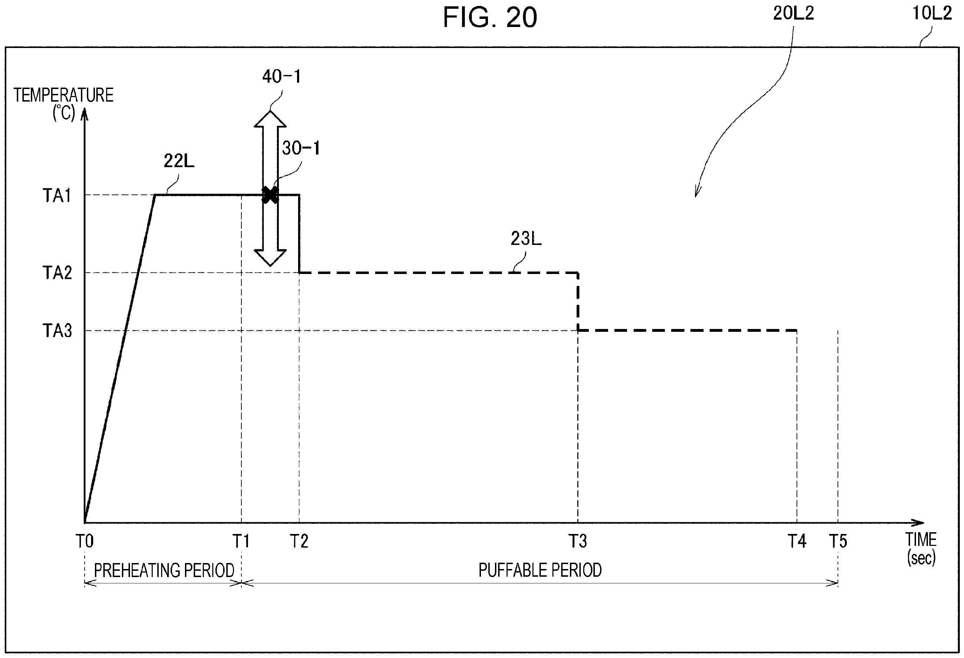

Fig. 20 is a diagram illustrating an example of the display image generated by the user terminal 200 according to the present embodiment. A display image 10L2 illustrated in Fig. 20 includes a graph 20L2, a line 22L, a line 23L, and a point 30, and such elements have features similar to those of the elements having the corresponding reference numerals illustrated in Fig. 7. That is, the display image 10L2 includes the line 22L indicating a heating profile from the start of preheating to a section that has already elapsed, and the line 23L indicating a heating profile of a section that has not yet elapsed. Further, the display image 10L2 displays the line 22L indicating the heating profile from the start of preheating to the section that has already elapsed in association with a point 30-1 indicating a value of the parameter at a puff detection time detected from the start time point to the current time point. The user can change the value of the parameter at the point 30-1 within a changeable range indicated by an arrow 40-1.

In the display image according to the third display example, a value of the parameter at a puff detection time is displayed in real time. This allows the user to customize the heating profile in real time while performing a puff action.

### - Fourth display example

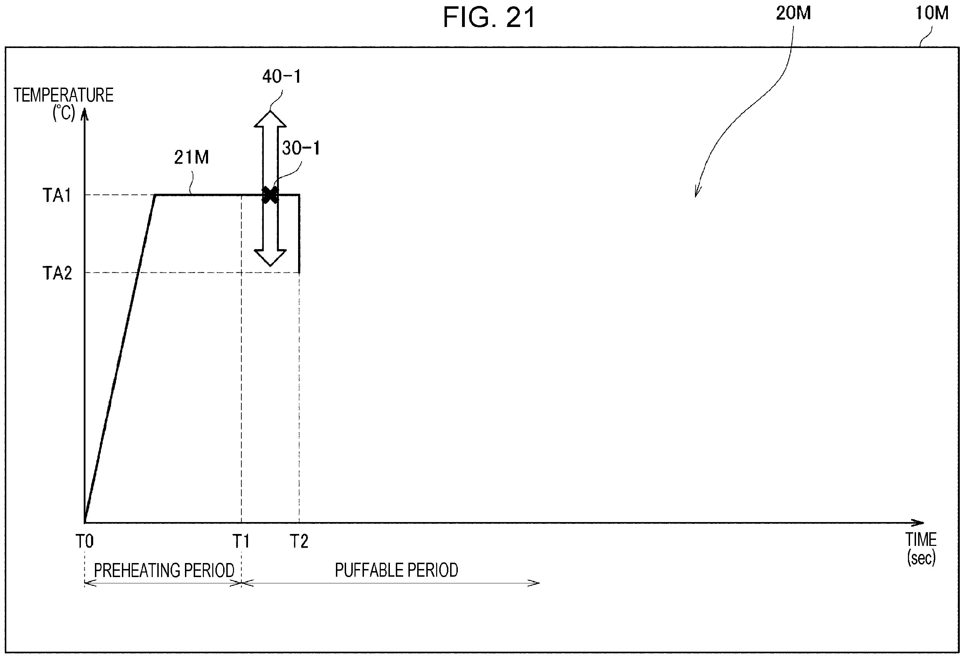

Fig. 21 is a diagram illustrating an example of the display image generated by the user terminal 200 according to the present embodiment. A display image 10M illustrated in Fig. 21 includes a graph 20M, a line 21M, and a point 30, and such elements have features similar to those of the elements having the corresponding reference numerals illustrated in Fig. 8. That is, the display image 10M displays the line 21M indicating a heating profile from the start time point to the current time point in association with a point 30-1 indicating a value of the parameter at a puff detection time detected from the start time point to the current time point. The user can change the value of the parameter at the point 30-1 within a changeable range indicated by an arrow 40-1.

In the display image according to the fourth display example, the time-series transition of the parameter for the heating profile and a value of the parameter at a puff detection time is displayed in real time. This allows the user to customize the heating profile in real time while performing a puff action.

### - Fifth display example

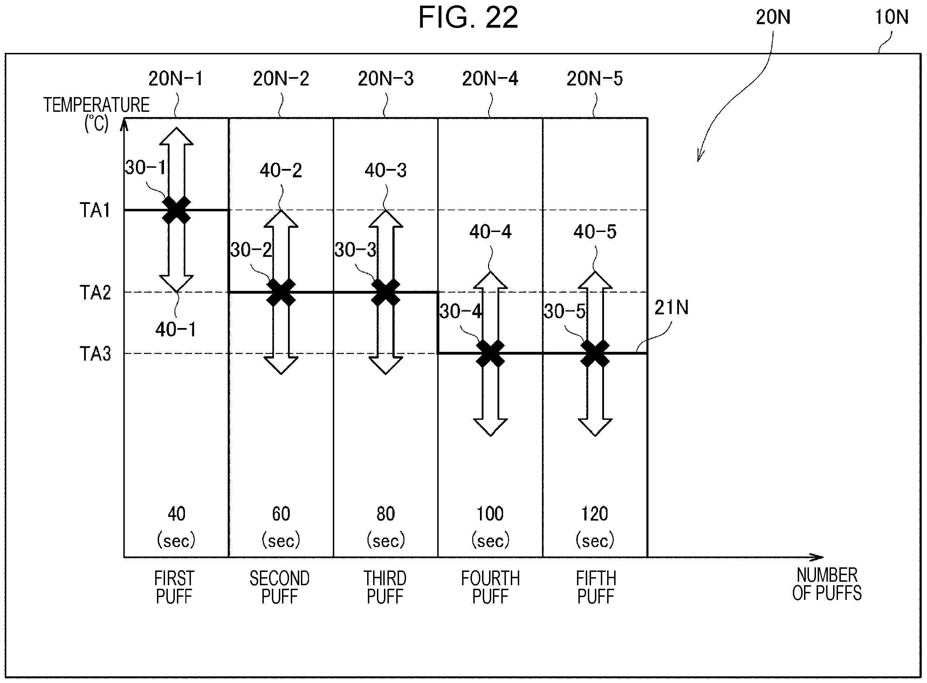

Fig. 22 is a diagram illustrating an example of the display image generated by the user terminal 200 according to the present embodiment. A display image 10N illustrated in Fig. 22 includes a graph 20N (20N-1 to 20N-5), a line 21N, and points 30, and such elements have features similar to those of the elements having the corresponding reference numerals illustrated in Fig. 9. That is, in the display image 10N, the line 21N indicating a heating profile in part of the entire time section from the start time point to the end time point, the part including puff detection times, is extracted and displayed as graphs 20N-1 to 20N-5. The user can change the values of the parameter at the points 30-1 to 30-5 within changeable ranges indicated by arrows 40-1 to 40-5 in the graphs 20N-1 to 20N-5, respectively.

The fifth display example allows the user to customize the heating profile with a focus on the puff detection times.

### - Sixth display example

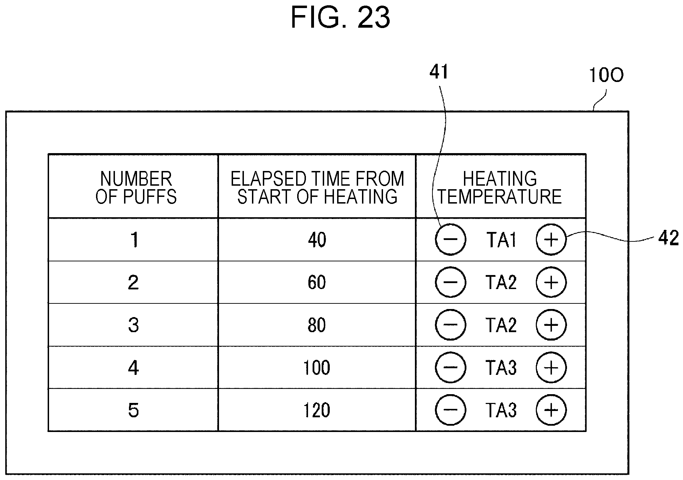

Fig. 23 is a diagram illustrating an example of the display image generated by the user terminal 200 according to the present embodiment. A display image 10O illustrated in Fig. 23 includes a table having features similar to those of the table illustrated in Fig. 10. That is, the display image 10O displays information related to puff detection times as a table. More specifically, the display image 10O includes a table indicating the number of puffs, an elapsed time from the start of heating (i.e., the start of preheating), and a heating temperature (i.e., a target temperature of the heater 121) at a puff detection time detected in the period from the start of preheating to the end of the puffable period. The user can operate a minus icon 41 to decrease the heating temperature. The user can operate a plus icon 42 to increase the heating temperature. The user interface (UI) for the parameter operation is not limited to the minus icon 41 and the plus icon 42. For example, a so-called scroll UI may be used such that the parameter can be increased or decreased by touching the parameter and scrolling the parameter horizontally or vertically.

The sixth display example allows the user to customize the heating profile with a focus on the puff detection times.

### - Seventh display example

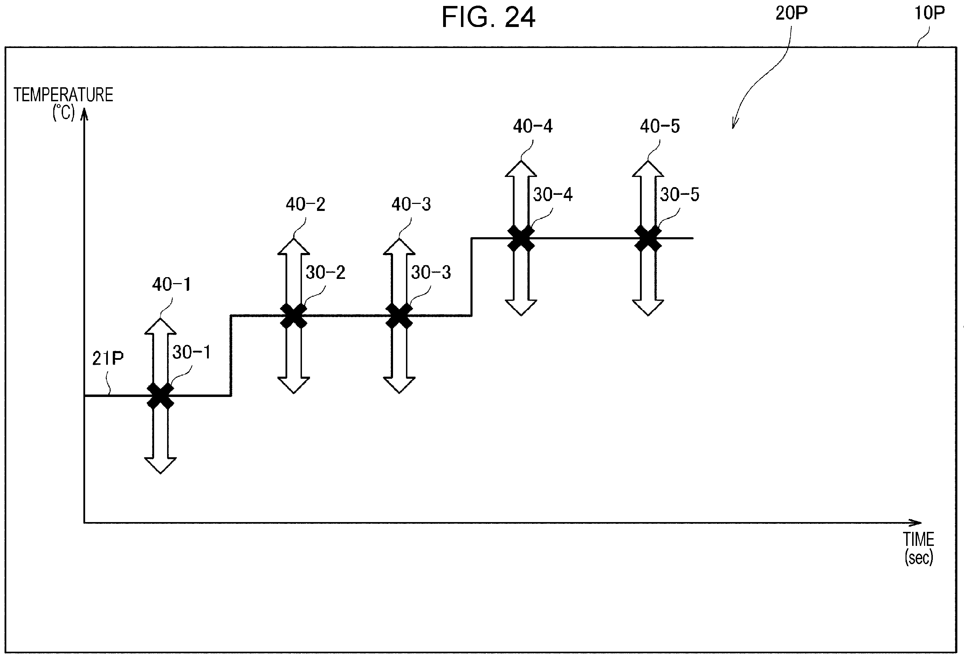

Fig. 24 is a diagram illustrating an example of the display image generated by the user terminal 200 according to the present embodiment. A display image 10P illustrated in Fig. 24 includes a graph 20P, a line 21P, and points 30, and such elements have features similar to those of the elements having the corresponding reference numerals illustrated in Fig. 11. That is, the display image 10P is a display image related to the inhaler device 100 according to the first configuration example, and includes the graph 20P depicting the line 21P indicating a heating profile from attachment of a new substrate to removal of the new substrate. The user can change the values of the parameter at the points 30-1 to 30-5 within changeable ranges indicated by arrows 40-1 to 40-5, respectively.

The display image related to the inhaler device 100 according to the seventh configuration example may be displayed in a manner similar to that of the display images according to the second to sixth display examples described above, and the parameter to be operated may be changed in accordance with the user operation.

### <2.4. Modifications of display image>

The display image may display, as the heating profile, a difference between the heating profile and another heating profile serving as a reference (hereinafter also referred to as a reference heating profile). The user performs an operation of changing the difference. Then, the user terminal 200 changes the heating profile to a heating profile having a difference designated by the user relative to the reference heating profile. This configuration allows the user to customize the heating profile using the reference heating profile as a reference.

The heating profile serving as a reference may be a heating profile on which the change has not been made. This configuration allows the user to customize the heating profile using a heating profile on which the change has not been made as a clue.

As an alternative, the heating profile serving as a reference may be a standard heating profile. This configuration allows the user to customize the heating profile using a standard heating profile as a clue.

### (1) Basic display image and customization

The following describes an example of customization of a basic display image corresponding to the first display example.

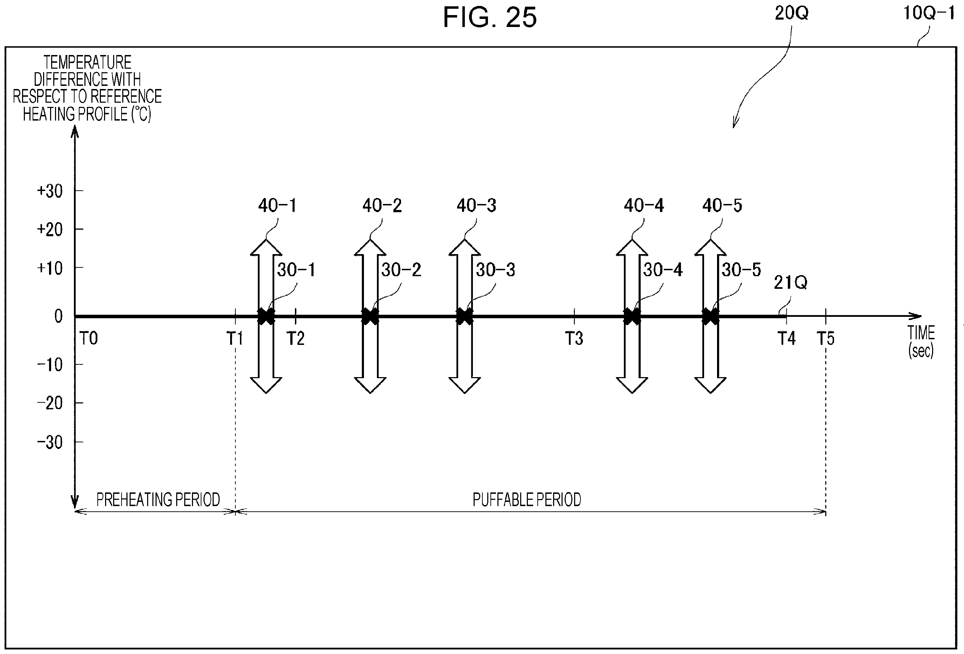

Fig. 25 is a diagram illustrating an example of the display image generated by the user terminal 200 according to the present embodiment. A display image 10Q-1 illustrated in Fig. 25 includes a graph 20Q depicting a line 21Q indicating a time-series change of a difference between a heating profile and a reference heating profile from the start of preheating to the end of the puffable period. In the graph 20Q, the horizontal axis represents time. In the graph 20Q, the vertical axis represents the difference between the parameter for the heating profile (i.e., the target temperature of the heater 121) and the parameter for the reference heating profile. Values of points 30 on the horizontal axis correspond to puff detection times. Values of the points 30 on the vertical axis indicate temperature differences of the heating profile from the reference heating profile at the puff detection times. In the example illustrated in Fig. 25, the line 21Q is always 0. That is, the heating profile is the same as the reference heating profile.

The parameter to be operated may be a value of the parameter at a puff detection time. The user performs an operation of selecting one of the points 30 indicating the values of the parameter at the puff detection times and shifting the selected point 30 up and down. The user terminal 200 changes the value of the parameter corresponding to the selected point 30 in accordance with the user operation. Specifically, the user terminal 200 changes the heating profile to a heating profile having a difference designated by the user operation relative to the reference heating profile. This configuration allows the user to customize the heating profile while referring to the feeling of inhaling the aerosol, based on the reference heating profile.

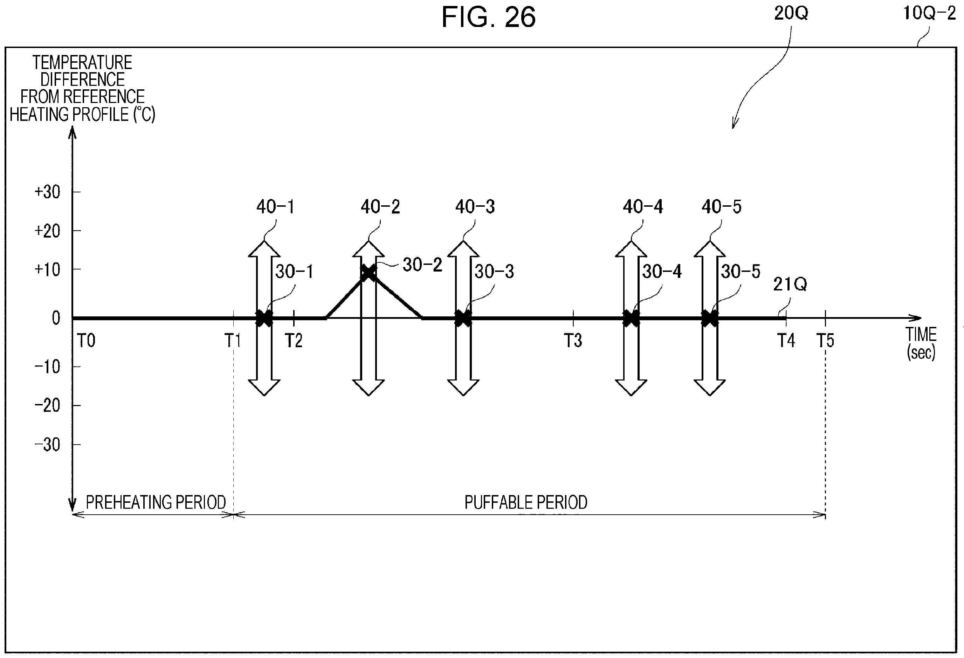

In an example, in the display image 10Q-1 illustrated in Fig. 25, a point 30-2 is selected and an operation of increasing the parameter is performed to produce a changed display image 10Q-2, which is illustrated in Fig. 26. Fig. 26 is a diagram illustrating an example of the display image generated by the user terminal 200 according to the present embodiment. As illustrated in Fig. 26, the temperature difference from the reference heating profile at the point 30-2 is increased to plus 10 from the state illustrated in Fig. 25. Accordingly, the target temperature at the point 30-2 of the heating profile is changed to a temperature obtained by adding 10°C to the target temperature in the reference heating profile at the corresponding time. In addition, the shape of a portion of the line 21Q at and around the point 30-2 is changed from a straight-line shape in Fig. 25 to an inverted V shape with the point 30-2 as a peak in Fig. 26. In this way, the user terminal 200 may also change a value of the parameter at another time that is continuous, in the time axis direction, with the time at which the change is made by the user operation.

The display image 10Q-1 includes arrows 40. The features of the arrows 40 are as described above with reference to Fig. 12 and the like.

As in the first display example, the display image 10Q-1 described above is displayed after the puffable period ends, that is, after the user finishes taking a series of puffs. Accordingly, the user can customize the heating profile while collectively recognizing the relationship between the heating profile for the series of puffs and the puff detection times.

### (2) Variations of display image and customization

### - First variation

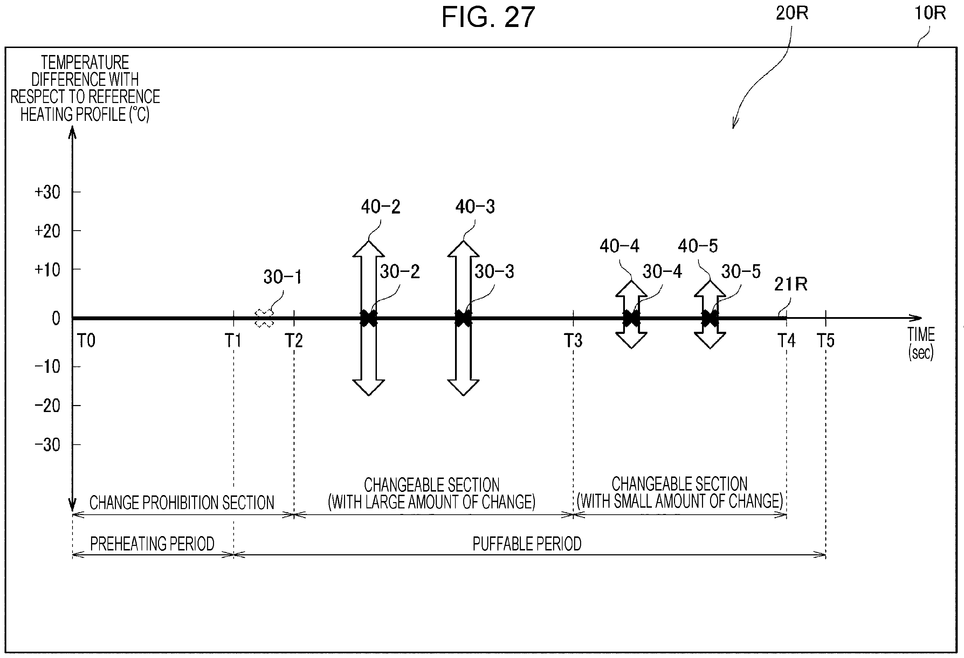

The first variation described above with reference to Fig. 14 is also applicable to the present modification. A first variation in the present modification will be described with reference to Fig. 27.

Fig. 27 is a diagram illustrating an example of the display image generated by the user terminal 200 according to the present embodiment. A display image 10R illustrated in Fig. 27 includes a graph 20R, a line 21R, points 30, and arrows 40, and such elements have features similar to those of the elements having the corresponding reference numerals illustrated in Fig. 25. The change prohibition section from the time T0 to the time T2, the changeable section (with a large amount of change) from the time T2 to the time T3, and the changeable section (with a small amount of change) from the time T3 to the time T4 are as described above with reference to Fig. 14. This configuration makes it possible to customize the heating profile within a range in which an aerosol is appropriately generated.

### - Second variation

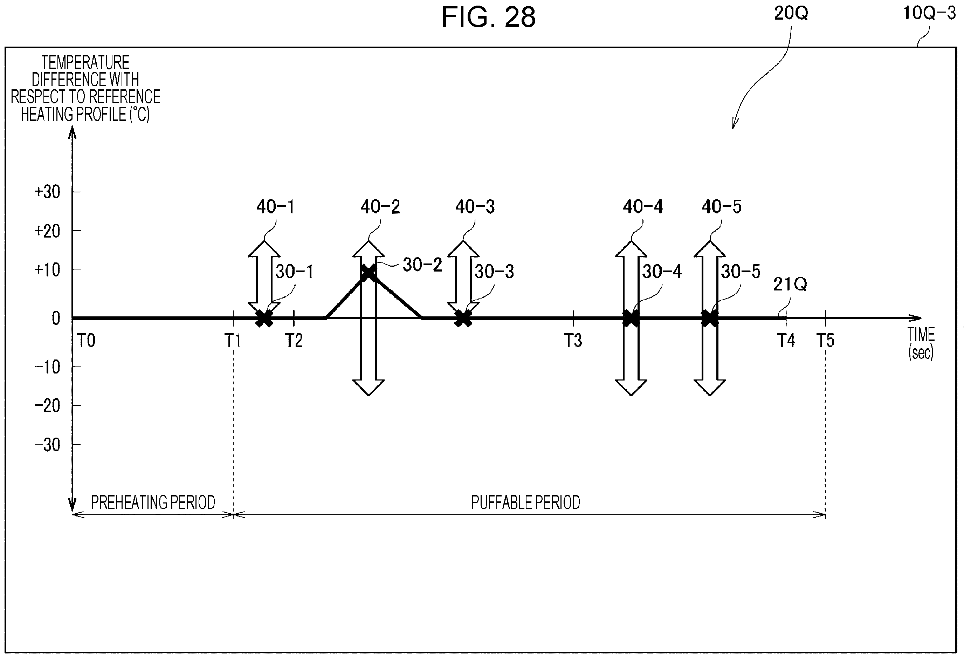

The second variation described above with reference to Fig. 15 is also applicable to the present modification. A second variation in the present modification will be described hereinafter. In an example, in the display image 10Q-1 illustrated in Fig. 25, the point 30-2 is selected and an operation of increasing the parameter is performed to produce a changed display image 10Q-3, which is illustrated in Fig. 28.

Fig. 28 is a diagram illustrating an example of the display image generated by the user terminal 200 according to the present embodiment. A graph 20Q illustrated in Fig. 28 has a shape similar to that of the graph 20Q illustrated in Fig. 26. Further, the values of the parameter at the point 30-1 and the point 30-3, which are continuous with the point 30-2 in the time axis direction, are changeable in a range that is reduced such that the values of the parameter can only be increased. In this way, the range in which the values of the parameter at the point 30-1 and the point 30-3 are changeable is changed in accordance with the change of the value of the parameter at the point 30-2. This configuration makes it possible to customize the heating profile within a range in which an aerosol is appropriately generated.

### - Third variation

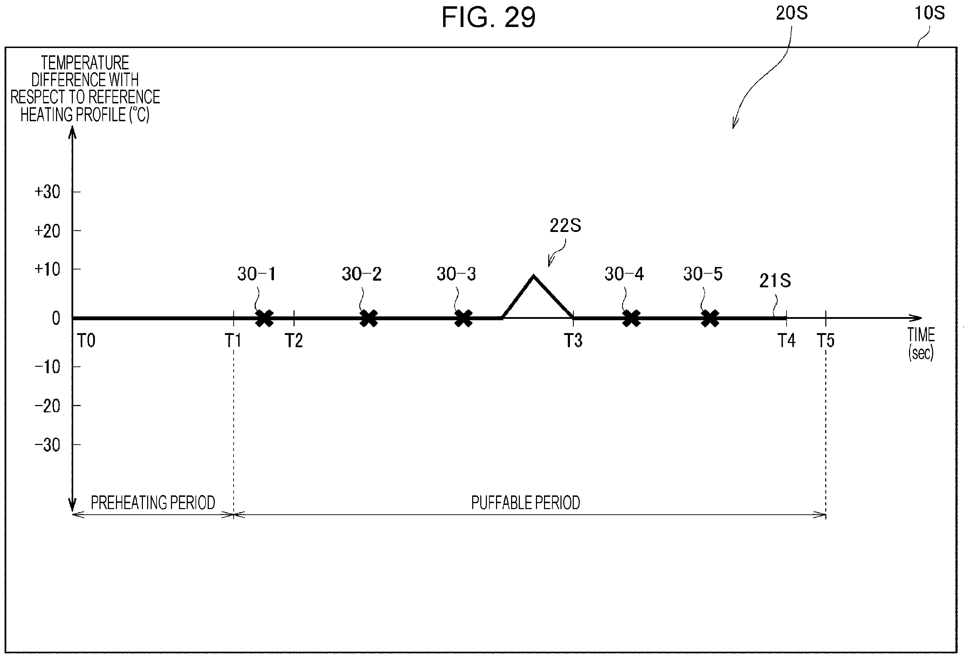

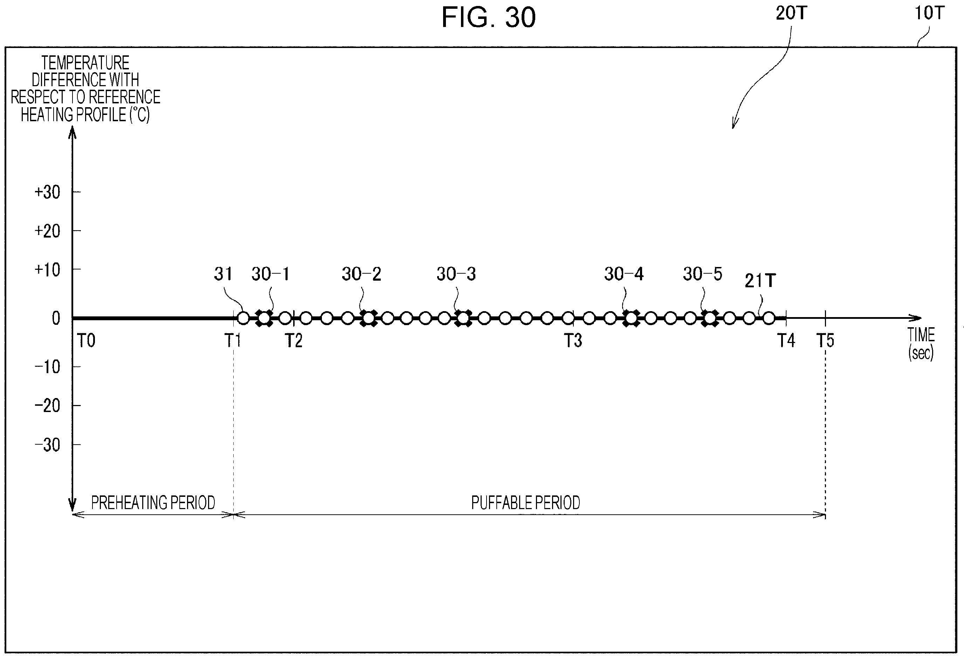

The third variation described above with reference to Fig. 16 and Fig. 17 is also applicable to the present modification. A third variation in the present modification will be described with reference to Fig. 29 and Fig. 30.

Fig. 29 is a diagram illustrating an example of the display image generated by the user terminal 200 according to the present embodiment. A display image 10S illustrated in Fig. 29 includes a graph 20S, a line 21S, and points 30, and such elements have features similar to those of the elements having the corresponding reference numerals illustrated in Fig. 25. The user may directly change the line 21S as an operation of changing a value of the parameter at a time other than a puff detection time. Fig. 29 illustrates a heating profile in which a portion of the line 21S indicating the heating profile, which is represented by reference numeral 22S, has been selected and an operation of increasing the parameter has been performed. This configuration makes it possible to achieve more flexible customization.

Fig. 30 is a diagram illustrating an example of the display image generated by the user terminal 200 according to the present embodiment. A display image 10T illustrated in Fig. 30 includes a graph 20T, a line 21T, and points 30, and such elements have features similar to those of the elements having the corresponding reference numerals illustrated in Fig. 25. In Fig. 30, a point 31 indicating a parameter to be operated is included in addition to the points 30 indicating values of the parameter at puff detection times. The user selects the point 31 and performs an operation of shifting the point 31 up and down. The user terminal 200 changes the value of the parameter corresponding to the selected point 31 in accordance with the user operation. This configuration makes it possible to improve visibility of a customizable parameter.

### - Fourth variation

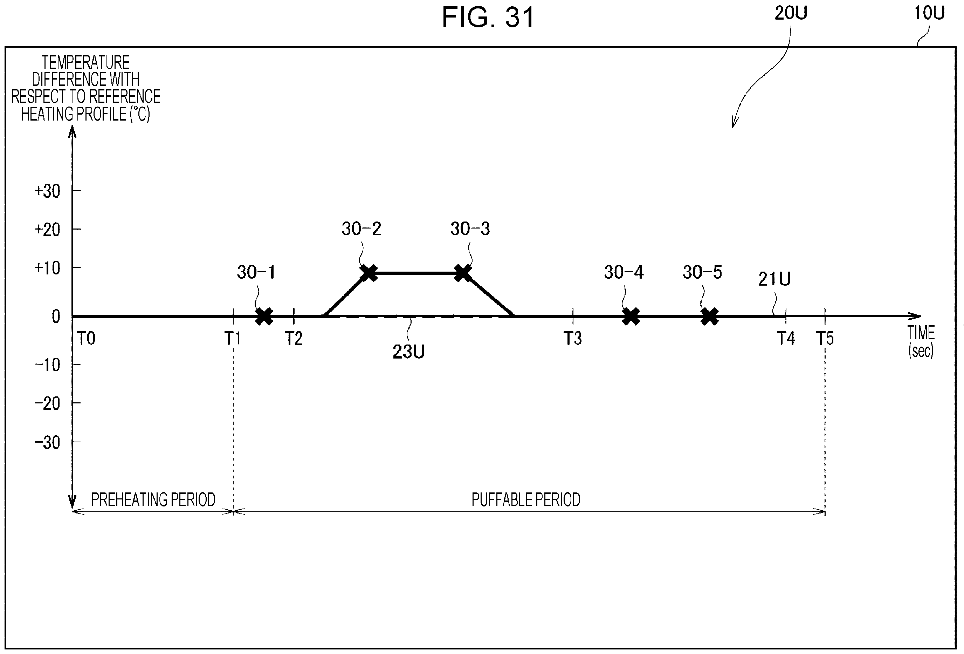

The fourth variation described above with reference to Fig. 18 is also applicable to the present modification. A fourth variation in the present modification will be described with reference to Fig. 31.

Fig. 31 is a diagram illustrating an example of the display image generated by the user terminal 200 according to the present embodiment. A display image 10U illustrated in Fig. 31 includes a graph 20U, a line 21U, a line 23U, and points 30, and such elements have features similar to those of the elements having the corresponding reference numerals illustrated in Fig. 25. In Fig. 31, the line 23U indicating a heating profile to be compared is displayed together with the line 21U indicating a heating profile on which a change has been made by the user. This configuration allows the user to customize the heating profile while recognizing the difference between the line 23U and the line 21U.

### - Fifth variation

Also in the present modification, as in the second display example described above with reference to Fig. 19 and the third display example described above with reference to Fig. 21, the parameter change operation may be received with the heating profile being displayed in the display image in real time.

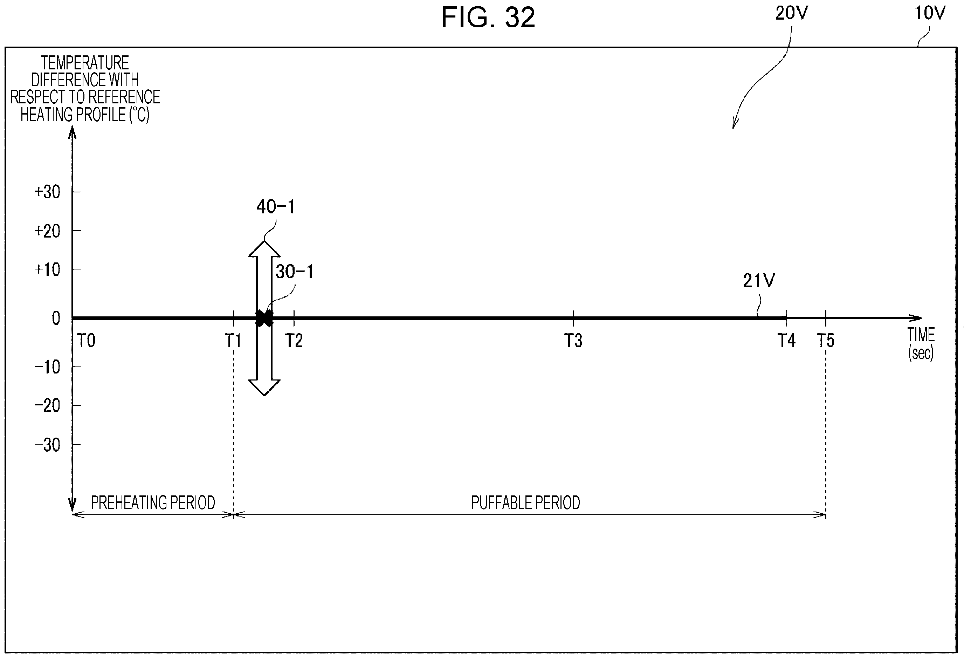

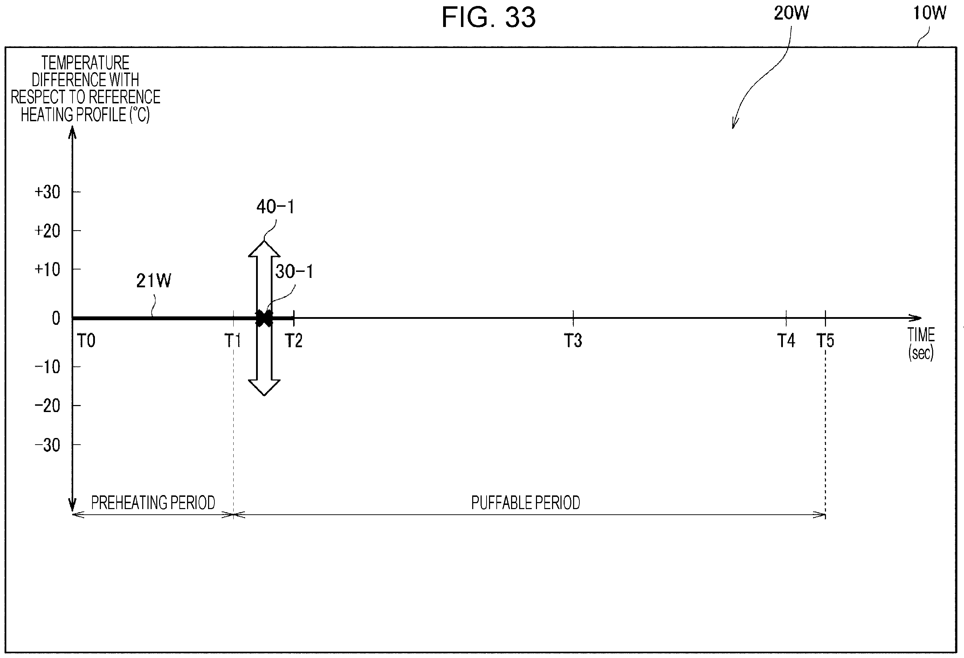

This variation will be described hereinafter with reference to Fig. 32 and Fig. 33.

Fig. 32 is a diagram illustrating an example of the display image generated by the user terminal 200 according to the present embodiment. A display image 10V illustrated in Fig. 32 includes a graph 20V, a line 21V, a point 30, and an arrow 40, and such elements have features similar to those of the elements having the corresponding reference numerals illustrated in Fig. 25. However, the display image 10V displays the line 21V indicating a heating profile from the start time point to the end time point in association with a point 30-1 indicating a value of the parameter at a puff detection time detected from the start time point to the current time point. The user can change the value of the parameter at the point 30-1 within a changeable range indicated by an arrow 40-1. This allows the user to customize the heating profile in real time while performing a puff action.

Fig. 33 is a diagram illustrating an example of the display image generated by the user terminal 200 according to the present embodiment. A display image 10W illustrated in Fig. 33 includes a graph 20W, a line 21W, a point 30, and an arrow 40, and such elements have features similar to those of the elements having the corresponding reference numerals illustrated in Fig. 25. However, the display image 10W displays the line 21W indicating a heating profile from the start time point to the current time point in association with a point 30-1 indicating a value of the parameter at a puff detection time detected from the start time point to the current time point. The user can change the value of the parameter at the point 30-1 within a changeable range indicated by an arrow 40-1. This allows the user to customize the heating profile in real time while performing a puff action.

### <2.5. Process flow>

### - Display of display image

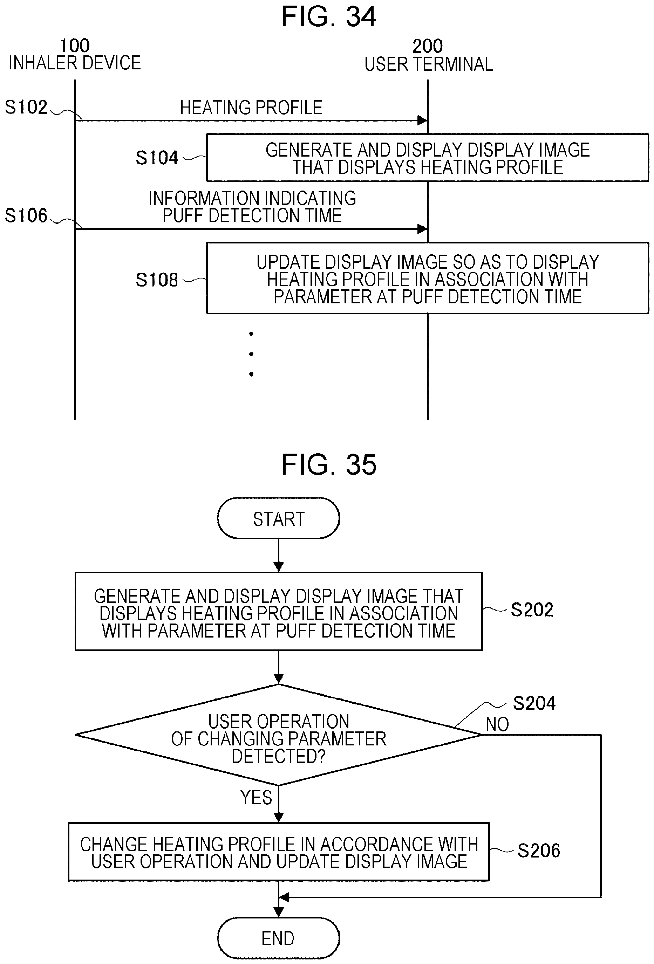

Fig. 34 is a sequence diagram illustrating an example of a process flow executed in the system 1 according to the present embodiment. As illustrated in Fig. 34, the inhaler device 100 and the user terminal 200 are involved in this sequence.

First, the inhaler device 100 transmits a heating profile to be used to generate an aerosol (step S102). Upon receipt of the heating profile, the user terminal 200 generates a display image that displays the received heating profile, and displays the display image (step S104).

Each time the inhaler device 100 detects a puff taken by the user, the inhaler device 100 transmits information indicating a puff detection time (step S106). Each time the user terminal 200 receives information indicating a puff detection time, the user terminal 200 updates the display image so that the heating profile is displayed in association with the parameter at the puff detection time (step S 108). The system 1 repeats the processing of step S106 and step S108 each time the user takes a puff.

### - Customization of heating profile

Fig. 35 is a flowchart illustrating an example of a process flow executed in the system 1 according to the present embodiment.

As illustrated in Fig. 35, first, the user terminal 200 generates a display image that displays a heating profile in association with a parameter at a puff detection time, and displays the display image (step S202).

Then, the user terminal 200 determines whether a user operation of changing the parameter is detected (step S204). If it is determined that the user operation of changing the parameter is detected (step S204: YES), the user terminal 200 changes the heating profile in accordance with the user operation and updates the display image so that the changed heating profile is displayed (step S206). Then, the process ends. If it is determined that the user operation of changing the parameter is not detected (step S204: NO), the process ends.

### «3. Supplementary note»

While a preferred embodiment of the present invention has been described in detail with reference to the accompanying drawings, the present invention is not limited to this example. It will be apparent that those skilled in the art to which the present invention belongs can achieve various modifications or variations without departing from the scope of the technical concept presented in the claims, and it is understood that such modifications or variations also fall within the technical scope of the present invention.

For example, in the embodiment described above, an example has been described in which the parameter for the heating profile is the temperature of the heater 121. However, the present invention is not limited to this example. In an example, the parameter may be a temperature of a portion heated by the heater 121. Examples of the portion heated by the heater 121 include the holder 140. In this case, the controller 116 controls supply of electric power to the heater 121 so that a temperature similar to a target temperature defined in the heating profile is achieved in the holder 140. In another example, the parameter may be information related to electricity to be supplied to the heater 121. For example, the parameter may be a voltage, a current, a resistance, or an electric power to be supplied to the heater 121. In this case, the controller 116 controls supply of electric power to the heater 121 so that a voltage, a current, a resistance, or an electric power similar to the voltage, the current, the resistance, or the electric power defined in the heating profile is supplied to the heater 121.

The heating profile described above is an example of a profile that is information related to an operation of generating an aerosol, which is performed by the inhaler device 100. The user terminal 200 may generate a display image that displays a profile other than the heating profile and information indicating a puff detection time in association with each other. An example of the profile other than the heating profile may be information indicating a result of an operation of generating an aerosol, which is performed by the inhaler device 100 (corresponding to a second profile; hereinafter also referred to as an operation result profile). The parameter for the operation result profile is information detected in response to the inhaler device 100 performing an operation of generating an aerosol. For example, while the heating profile is information indicating a target temperature transition, the operation result profile may be information indicating an actual temperature transition. This configuration allows the user to recognize the relationship between the operation result profile and a puff detection time while referring to the feeling of inhaling the aerosol.

Like the heating profile, examples of the parameter for the operation result profile include information related to the temperature of the heater 121, the temperature of a portion heated by the heater 121, and electricity to be supplied to the heater 121. Other examples of the parameter for the operation result profile may include an amount of aerosol generated by the heater 121 and to be inhaled by the user (hereinafter also referred to as an amount of aerosol delivery). In this case, the sensor 112 includes, as a sensor for detecting the amount of aerosol delivery, a filter for collecting the aerosol and a component analyzer for analyzing a component of the collected aerosol. The amount of aerosol delivery may be an amount of main aerosol component per puff action, which is delivered into the oral cavity of the user. The main aerosol component is a visible aerosol component generated when various aerosol sources contained in the substrate are heated at a predetermined temperature or higher. The aerosol sources contained in the substrate are typically propylene glycol and glycerine. In a case where the substrate contains a flavor source such as tobacco, an aerosol component derived from the flavor source is also included in the main aerosol component.

For example, the display image may further include information predicted to be detected when the inhaler device 100 operates in accordance with a heating profile on which a change has been made. Examples of the predicted information include an operation result profile. In an example, the user terminal 200 learns a correspondence relationship between a heating profile and an operation result profile related to an amount of aerosol delivery. Then, the user terminal 200 may refer to the learning result, predict the amount of aerosol delivery when the inhaler device 100 operates in accordance with the heating profile on which the change has been made, and display the predicted amount of aerosol delivery in the display image. This configuration allows customization with reference to a predicted amount of aerosol delivery, making it possible to more easily search for a heating profile that achieves desired smoking quality.

For example, the operation result profile may be customized. In this case, the user terminal 200 generates a heating profile for implementing the changed operation result profile, and controls the inhaler device 100 so that the inhaler device 100 can operate in accordance with the generated heating profile. In an example, the user terminal 200 learns a correspondence relationship between a heating profile and an operation result profile related to an amount of aerosol delivery. Then, the user terminal 200 refers to the learning result and generates a heating profile for implementing an operation result profile related to an amount of aerosol delivery on which a change has been made. This configuration allows direct customization of the amount of aerosol delivery, making it possible to more easily search for a heating profile that achieves desired smoking quality.

The devices described herein may be implemented as independent devices, and all or some of them may be implemented as separate devices. For example, in the functional configuration example of the user terminal 200 illustrated in Fig. 3, the function of generating a display image and the function of changing a heating profile in accordance with a user operation, which are included in the controller 250, may be provided for a device such as a server connected to the user terminal 200 via a network or the like. Furthermore, the function of generating a display image and the function of changing a heating profile in accordance with a user operation, which are included in the controller 250, may be provided for the inhaler device 100.

A series of processes performed by each of the devices described herein may be implemented using any one of software, hardware, and a combination of software and hardware. A program constituting the software is stored in advance in, for example, an internal or external recording medium (non-transitory medium) of each device. For example, each program is read into a RAM at the time of execution by a computer and executed by a processor such as a CPU. Examples of the recording medium include a magnetic disk, an optical disk, a magneto-optical disk, and a flash memory. Further, the computer program described above may be distributed via, for example, a network without using a recording medium.

The processes described herein using the flowchart and the sequence diagram may not necessarily be executed in the illustrated order. Some processing steps may be executed in parallel. Any additional processing step may be used, or some processing steps may be omitted.

The following configurations also fall within the technical scope of the present invention.
(1) An information processing device including a controller configured to:
   generate a display image that displays a profile, the profile being information indicating a time-series change of a parameter related to an operation of generating an aerosol, the operation being performed by an inhaler device configured to generate the aerosol by using a substrate; and
   change the profile and display of the profile in the display image in accordance with a user operation on the parameter to be operated in the generated display image.
(2) The information processing device according to (1) above, wherein
   the controller is configured to change the parameter to be operated in the profile in accordance with the user operation of changing the parameter to be operated.
(3) The information processing device according to (2) above, wherein
   the display image includes information indicating that the parameter to be operated is changeable.
(4) The information processing device according to (2) or (3) above, wherein
   the display image includes information indicating a range in which the parameter to be operated is changeable.
(5) The information processing device according to any one of (2) to (4) above, wherein
   the controller is configured to change a range in which the parameter to be operated is changeable, in response to another parameter to be operated being changed in accordance with the user operation.
(6) The information processing device according to any one of (2) to (5) above, wherein
   the parameter to be operated includes the parameter at a puff detection time, the puff detection time being a time at which inhalation of the aerosol generated by the inhaler device is detected, the inhalation being performed by a user.
(7) The information processing device according to any one of (2) to (6) above, wherein
   the parameter to be operated includes the parameter at a time other than a puff detection time, the puff detection time being a time at which inhalation of the aerosol generated by the inhaler device is detected, the inhalation being performed by a user.
(8) The information processing device according to any one of (2) to (7) above, wherein
   the display image displays the parameter to be operated in a mode different from a mode of a parameter that is not to be operated.
(9) The information processing device according to any one of (2) to (8) above, wherein
   the display image displays the profile from a start time point to an end time point in association with information indicating the parameter to be operated from the start time point to the end time point.
(10) The information processing device according to any one of (2) to (8) above, wherein
   the display image displays the profile from a start time point to an end time point in association with information indicating the parameter to be operated from the start time point to a current time point.
(11) The information processing device according to any one of (2) to (8) above, wherein
   the display image displays the profile from a start time point to a current point in association with information indicating the parameter to be operated from the start time point to the current time point.
(12) The information processing device according to any one of (2) to (8) above, wherein
   the display image displays an extracted portion of the profile in part of an entire time section from a start time point to an end time point, the part including a puff detection time, the puff detection time being a time at which inhalation of the aerosol generated by the inhaler device is detected, the inhalation being performed by a user.
(13) The information processing device according to any one of (1) to (12) above, wherein
   the display image further displays another profile to be compared with the profile.
(14) The information processing device according to any one of (1) to (13) above, wherein
   the display image displays, as the profile, a difference between the profile and another profile serving as a reference.
(15) The information processing device according to (13) or (14) above, wherein
   the other profile is the profile on which no change has been made.
(16) The information processing device according to any one of (1) to (15) above, wherein
   the profile is a first profile including, as the parameter, information that defines an operation in which the inhaler device generates the aerosol.
(17) The information processing device according to (16) above, wherein
   the controller is configured to control the inhaler device so that the inhaler device operates in accordance with the first profile on which a change has been made.
(18) The information processing device according to (16) or (17) above, wherein
   the display image further includes information predicted to be detected in response to the inhaler device operating in accordance with the first profile on which a change has been made.
(19) The information processing device according to any one of (1) to (18) above, wherein
   the profile is a second profile including, as the parameter, information detected in response to the inhaler device performing the operation of generating the aerosol.
(20) The information processing device according to (19) above, wherein
   the controller is configured to generate a first profile including, as a parameter, information that defines an operation in which the inhaler device generates the aerosol to implement the second profile on which a change has been made, and control the inhaler device so that the inhaler device operates in accordance with the generated first profile.
(21) The information processing device according to any one of (1) to (20) above, wherein
   the display image displays the profile as a graph.
(22) The information processing device according to any one of (1) to (21) above, wherein
   the display image displays, as a table, the parameter to be operated.
(23) An information processing method including:
   generating a display image that displays a profile, the profile being information indicating a time-series change of a parameter related to an operation of generating an aerosol, the operation being performed by an inhaler device configured to generate the aerosol by using a substrate; and
   changing the profile and display of the profile in the display image in accordance with a user operation on the parameter to be operated in the generated display image.
(24) A program for causing a computer to function as a controller configured to:
   generate a display image that displays a profile, the profile being information indicating a time-series change of a parameter related to an operation of generating an aerosol, the operation being performed by an inhaler device configured to generate the aerosol by using a substrate; and
   change the profile and display of the profile in the display image in accordance with a user operation on the parameter to be operated in the generated display image.

### Reference Signs List

- 1: system
- 100: inhaler device
- 110: power supply unit
- 111: power supply
- 112: sensor
- 113: notifier
- 114: memory
- 115: communicator
- 116: controller
- 120: cartridge
- 121: heater
- 122: liquid guide
- 123: liquid storage
- 124: mouthpiece
- 130: flavor imparting cartridge
- 131: flavor source
- 140: holder
- 141: internal space
- 142: opening
- 143: bottom
- 144: heat insulator
- 150: stick substrate
- 151: substrate
- 152: inhalation port
- 180: airflow path
- 181: air inlet hole
- 182: air outlet hole
- 200: user terminal
- 210: input unit
- 220: output unit
- 230: communicator
- 240: memory
- 250: controller

## Claims

1. An information processing device comprising a controller configured to:
generate a display image that displays a profile, the profile being information indicating a time-series change of a parameter related to an operation of generating an aerosol, the operation being performed by an inhaler device configured to generate the aerosol by using a substrate; and
change the profile and display of the profile in the display image in accordance with a user operation on the parameter to be operated in the generated display image.

2. The information processing device according to claim 1, wherein
the controller is configured to change the parameter to be operated in the profile in accordance with the user operation of changing the parameter to be operated.

3. The information processing device according to claim 2, wherein
the display image includes information indicating that the parameter to be operated is changeable.

4. The information processing device according to claim 2 or 3, wherein
the display image includes information indicating a range in which the parameter to be operated is changeable.

5. The information processing device according to any one of claims 2 to 4, wherein
the controller is configured to change a range in which the parameter to be operated is changeable, in response to another parameter to be operated being changed in accordance with the user operation.

6. The information processing device according to any one of claims 2 to 5, wherein
the parameter to be operated includes the parameter at a puff detection time, the puff detection time being a time at which inhalation of the aerosol generated by the inhaler device is detected, the inhalation being performed by a user.

7. The information processing device according to any one of claims 2 to 6, wherein
the parameter to be operated includes the parameter at a time other than a puff detection time, the puff detection time being a time at which inhalation of the aerosol generated by the inhaler device is detected, the inhalation being performed by a user.

8. The information processing device according to any one of claims 2 to 7, wherein
the display image displays the parameter to be operated in a mode different from a mode of a parameter that is not to be operated.

9. The information processing device according to any one of claims 2 to 8, wherein
the display image displays the profile from a start time point to an end time point in association with information indicating the parameter to be operated from the start time point to the end time point.

10. The information processing device according to any one of claims 2 to 8, wherein
the display image displays the profile from a start time point to an end time point in association with information indicating the parameter to be operated from the start time point to a current time point.

11. The information processing device according to any one of claims 2 to 8, wherein
the display image displays the profile from a start time point to a current point in association with information indicating the parameter to be operated from the start time point to the current time point.

12. The information processing device according to any one of claims 2 to 8, wherein
the display image displays an extracted portion of the profile in part of an entire time section from a start time point to an end time point, the part including a puff detection time, the puff detection time being a time at which inhalation of the aerosol generated by the inhaler device is detected, the inhalation being performed by a user.

13. The information processing device according to any one of claims 1 to 12, wherein
the display image further displays another profile to be compared with the profile.

14. The information processing device according to any one of claims 1 to 13, wherein
the display image displays, as the profile, a difference between the profile and another profile serving as a reference.

15. The information processing device according to claim 13 or 14, wherein
the other profile is the profile on which no change has been made.

16. The information processing device according to any one of claims 1 to 15, wherein
the profile is a first profile including, as the parameter, information that defines an operation in which the inhaler device generates the aerosol.

17. The information processing device according to claim 16, wherein
the controller is configured to control the inhaler device so that the inhaler device operates in accordance with the first profile on which a change has been made.

18. The information processing device according to claim 16 or 17, wherein
the display image further includes information predicted to be detected in response to the inhaler device operating in accordance with the first profile on which a change has been made.

19. The information processing device according to any one of claims 1 to 18, wherein
the profile is a second profile including, as the parameter, information detected in response to the inhaler device performing the operation of generating the aerosol.

20. The information processing device according to claim 19, wherein
the controller is configured to generate a first profile including, as a parameter, information that defines an operation in which the inhaler device generates the aerosol to implement the second profile on which a change has been made, and control the inhaler device so that the inhaler device operates in accordance with the generated first profile.

21. The information processing device according to any one of claims 1 to 20, wherein
the display image displays the profile as a graph.

22. The information processing device according to any one of claims 1 to 21, wherein
the display image displays, as a table, the parameter to be operated.

23. An information processing method comprising:
generating a display image that displays a profile, the profile being information indicating a time-series change of a parameter related to an operation of generating an aerosol, the operation being performed by an inhaler device configured to generate the aerosol by using a substrate; and
changing the profile and display of the profile in the display image in accordance with a user operation on the parameter to be operated in the generated display image.

24. A program for causing a computer to function as a controller configured to:
generate a display image that displays a profile, the profile being information indicating a time-series change of a parameter related to an operation of generating an aerosol, the operation being performed by an inhaler device configured to generate the aerosol by using a substrate; and
change the profile and display of the profile in the display image in accordance with a user operation on the parameter to be operated in the generated display image.
